(19) 

Europäisches
Patentamt
European
Patent Office
Office européen
des brevets

(11) **EP 4 262 843 B1**

(12) **EUROPEAN PATENT SPECIFICATION**

(45) Date of publication and mention
of the grant of the patent:
**05.06.2024  Bulletin 2024/23**

(21) Application number: **21856906.9**

(22) Date of filing: **16.12.2021**

(51) International Patent Classification (IPC):
*A61K 38/16* (2006.01)       *A61K 38/51* (2006.01)
*A61P 31/04* (2006.01)       *C07K 14/315* (2006.01)

(52) Cooperative Patent Classification (CPC):
**A61P 31/04; C12N 9/80;** A61K 38/00;
C12R 2001/46; C12Y 305/01028

(86) International application number:
**PCT/SK2021/050016**

(87) International publication number:
**WO 2022/132059 (23.06.2022 Gazette 2022/25)**

(54) **ANTIMICROBIAL PROTEIN, ANTIMICROBIAL RECOMBINANT PROTEIN WITH LYTIC PROPERTIES, EXPRESSION VECTOR, METHOD OF THEIR PREPARATION AND USE**

ANTIMIKROBIELLES PROTEIN, ANTIMIKROBIELLES REKOMBINANTES PROTEIN MIT LYTISCHEN EIGENSCHAFTEN, EXPRESSIONSVEKTOR, VERFAHREN ZU DEREN HERSTELLUNG UND VERWENDUNG

PROTÉINE ANTIMICROBIENNE, PROTÉINE RECOMBINANTE ANTIMICROBIENNE À PROPRIÉTÉS LYTIQUES, VECTEUR D'EXPRESSION, LEUR PROCÉDÉ DE PRÉPARATION ET LEUR UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **17.12.2020   SK 500752020**

(43) Date of publication of application:
**25.10.2023   Bulletin 2023/43**

(73) Proprietors:
• **Ustav Molekularnej Biologie SAV, v. v. i.**
**845 51 Bratislava (SK)**
• **Univerzita Komenskeho V Bratislave**
**814 99 Bratislava (SK)**

(72) Inventors:
• **BUKOVSKA, Gabriela**
**841 02 Bratislava (SK)**
• **BOCANOVA, Lucia**
**919 42 Voderady (SK)**

• **HALGASOVA, Nora**
**841 01 Bratislava (SK)**
• **KAJSIKOVA, Maria**
**900 43 Hamuliakovo (SK)**
• **DRAHOVSKA, Hana**
**831 03 Bratislava (SK)**

(74) Representative: **Majlingová, Zuzana**
**Majlingová & Partners, s.r.o.**
**Budatínska 12**
**851 06 Bratislava (SK)**

(56) References cited:
**WO-A1-2016/184855        WO-A1-2019/213592
WO-A2-2010/149792        WO-A2-2017/125585
KR-A- 20100 035 879**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 262 843 B1

## Description

### Technical field

The present invention relates to the use of a novel endolysin isolated from the strain *Streptococcus agalactiae* and its use in the treatment, diagnosis and prophylaxis of infections caused by pathogenic species, in particular bacterial species of the genus Streptococcus.

The main problem of modern medicine is the rising growth and incidence of multidrug-resistant bacteria, especially due to the long-term and frequent use of antibiotics. One potential solution to alleviate the presence of multidrug-resistant bacteria is the use of phages and/or their protein products in human and veterinary therapy. Phage lysins have successfully been used to control pathogenic bacteria localised on the mucosa as well as in infected tissues in several animal models. Their specificity in killing bacteria without disturbing the normal microflora, as well as the low probability of bacterial resistance, make them ideal anti-infective agents.

### Background art

A potential problem in the use of endolysins is the development of antibodies by the immune system. Unlike antibiotics, which are small non-immunogenic molecules, endolysins are proteins that stimulate the host immune response in both systemic and mucosal delivery. However, studies on the immunogenicity of endolysins have shown that the immune response reduces the activity of endolysin but does not prevent bacterial killing.

The genus *Streptococcus consists* of Gram-positive cocci bacteria, including pathogens that have a serious impact on human health. *Streptococcus agalactiae* is an opportunistic pathogen that is part of the gastrointestinal and urogenital tracts in humans. The pathogenic bacteria *Streptococcus agalactiae* group B (GBS) causes serious diseases in humans, but are primarily known to cause infections in pregnant women and children. According to the World Health Organization, 15-45% of pregnant women suffer from *Streptococcus agalactiae* GBS colonisation, many of which are asymptomatic. In new-borns, the infection usually occurs after transmission from the vaginal flora of the mother to the foetus. GBS is a major cause of neonatal morbidity and mortality. If children become ill with group B streptococci, it is usually during the first weeks up to 3 months. Infection can cause blood poisoning (septicaemia), inflammation or damage to the lungs, or also neonatal meningitis and brain damage. In adults, especially those who have a weakened immune system, often due to other diseases, infection with group B streptococci can lead to other types of serious diseases, such as streptococcal toxic shock syndrome, septicaemia, urinary tract infections, pneumonia, meningitis, and endocarditis as well as necrotizing fasciitis.

In Slovakia a study was conducted over the period of 2013-2015 to determine the occurrence of *Streptococcus agalactiae* in women (Artimová, 2016). The average prevalence of *Streptococcus agalactiae* was 13.5% for women, 6.5% for pregnant women and 10.2% for neonates. The resistance of *Streptococcus agalactiae* to penicillin was 0% in samples from pregnant women and neonates; resistance to erythromycin averaged 59.8% in pregnant women, and 60.4% in neonates; resistance to clindamycin averaged 59.8% in pregnant women, and 53.3% in neonates; and resistance to tetracycline averaged 82.3%, and averaged 86.2% in strains isolated from neonates. (Artimová, 2016)

Classical treatment of infections caused by *Streptococcus agalactiae* consists in the administration of antibiotics, and the treatment of infections in pregnant women is based on antibiotic prophylaxis. Pregnant women are screened at 35 to 37 weeks of gestation, and women who are positive for GBS are decolonized before and during birth by the administration of antibiotics. A complication of the treatment is the increased resistance of these pathogens to the administered antibiotics, as well as the allergic reaction of the mother and her foetus to the administered drug. The estimated incidence of allergic reactions to penicillin treatment is 0.7% to 4% of all treated. The estimated risk of anaphylaxis is 4/10 000 to 4/100 000 recipients. Moreover, clinical studies have shown that the administration of antibiotics to pregnant women may be associated with adverse effects on the neonate, such as the development of necrotizing enterocolitis. Due to the disadvantages of treating GBS infections with antibiotics and the occurrence of recurrent infections, new treatment options are currently being sought. Phage therapy, which uses bacteriophages and their products as bioagents in the treatment of infectious diseases caused by bacteria, appears to be a suitable alternative. The proper treatment of infections requires the identification of a specific target pathogen as well as an effective lytic bacteriophage. Despite several *in vitro* studies of bacteriophages with lytic activity against human GBS isolates, so far only tempered bacteriophages have been identified in this organism, but these are not suitable for use in phage therapy. One of the treatment options for infections caused by *Streptococcus agalactiae* is therefore the use and application of phage endolysins. Endolysins are bacteriophage enzymes that serve to degrade the host cell peptidoglycan at the end of the bacteriophage lysis cycle, resulting in bacterial cell lysis and the release of phage virions into the environment.

The endolysins from bacteriophages infecting Gram-positive hosts studied so far have a predominantly two-domain modular protein structure: N-terminal catalytic domain and C-terminal binding domain. In some cases, mainly staphylococcal and streptococcal phage endolysins have two to three different catalytic domains attached to a single binding domain. We divide endolysins into five groups according to the localisation of the bond cleavage site in the peptidoglycan: (A) N-acetylmuramidase, (B) N-acetyl-β-D-glucosaminidase, (C) N-acetylmuramoyl-L-alanine amidase, (D) L-alanoyl-D-glutamate endopeptidase to (E) lytic transglycosylase. The C-terminal binding domain of most endolysins is characterised by a unique sequence that bears no resemblance to other known motifs responsible for cell wall binding. The characterised binding domains include, for example: a) GW domain b) PlyPSA domain c) FtsN- peptidoglycan binding domain; d) choline binding domain; e) LysM domain; and f) SH3 domain.

Strains of *S. agalactiae* often contain prophages in their genome, which also include many endolysins. Several endolysins with therapeutic potential in the treatment of group B streptococcal infections have currently been identified, which are, however, protected by international patents. The best known and best characterised endolysins effective against strains *Streptococcus agalactiae* are the endolysins B30, PlyGBS, LambdaSa1, LambdaSa2 and PlySK1249.

Endolysin B30 is derived from the tempered bacteriophage B30, which is found in the genome of the clinical isolate *Streptococcus agalactiae* GBS 3330. The bacteriophage B30 endolysin contains two catalytic domains, CHAP and a glycosyl hydrolase GH25, and a SH3b binding domain. Using phage lysin B30, the growth of several strains of Streptococcus agalactiae GBS is inhibited. The enzyme also lysed other β-hemolytic streptococci of groups A, C, E and G. It was also effective against strains of *Streptococcus* that cause mastitis in cattle, but also showed activity against lactic acid strains of *Streptococcus thermophilus* and *Lactococcus lactis subsp. cremoris.* Endolysin B30 is protected by patent US007572602B1.

The endolysin PlyGBS originates from prophage NCTC 11261 of clinical isolate GBS 11261. The gene for the endolysin *plygbs* was cloned into an expression system of *Escherichia coli,* and a recombinant endolysin was isolated by ion chromatography. PlyGBS contains two enzyme catalytic domains: endopeptidase and muramidase and an unidentified binding domain region. PlyGBS endolysin is active against group A, B, C, G, and L streptococci. Lytic activity of PlyGBS has also been detected *in vivo* on mice. It was confirmed in an animal mouse model that GBS colonisation, not only in the urogenital tract (UGT) but also in the pharynx, was reduced after a single dose of PlyGBS. The pH optimum for PlyGBS endolysin is ~ 5.0. This endolysin is specific for streptococci and does not show bacteriological activity against common vaginal microflora such as e.g. *Lactobacillus acidophilus* and *Lactobacillus crispatus.* It is suitable for the reducing vaginal GBS colonisation in pregnant women and for obstetric prophylaxis of GBS infections in neonates (Nelson et al., 2006). The authors also prepared recombinant endolysin mutants. They found that the PlyGBS90-1 mutant more reduced colonisation of *Streptococcus agalactiae* (GBS) in the vagina of the tested mice compared to the original PlyGBS endolysin. The PlyGBS endolysin mutant is protected by International Patent EP1917355B1.

Whole-genome sequencing of the strain *Streptococcus agalactiae* 2603 V/R revealed multiple genes for prophage lysins. Two of them, LambdaSa1 and LambdaSa2, were cloned and isolated. LambdaSa1 contains an amidase_5 catalytic domain and a SH3b binding domain. LambdaSa2 contains the catalytic domains: amidase_5 and amidase_4 and in the central region two Cpl-7 domains. Compared to lysin B30, isolated lysins showed clearer, although smaller zones on an agar plate with *Streptococcus agalactiae* cell substrates. The isolated enzymes lysed the cell walls of other bacteria as well: *Streptococcus pneumoniae* and *Staphylococcus aureus.* Patent WO2009143446A3 relates to a truncated form of the LambdaSa2 endolysin, effective against both streptococci and staphylococci.

The phage lysin PlySK1249 is encoded in the prophage region in the genome of the strain *Streptococcus dysgalactiae subsp. equimilis* SK1249. The protein contains the LysM binding domain located in the central region between the two enzymatic domains, amidase_3 and CHAP. Lysin PlySK1249 showed lytic activity against β-hemolytic streptococci (*Streptococcus agalactiae* group B, *Streptococcus pyogenes, Streptococcus dysgalactiae*). Endolysin PlySK1249 has been used in a mouse model for the treatment of GBS bacteraemia and has proven to be a suitable candidate for preclinical testing in the treatment of diseases caused by GBS infection (Patent US20160145591A1).

Two distinct endolysins, PlySs1 and its mutant PlySs2, were isolated from the prophage region of the strain *Streptococcus suis.* The endolysin protein PlySs1 contains a glucose-aminidase endopeptidase domain and, in the central part, two Cpl-7 domains. The PlySs2 protein contains a CHAP domain and a SH3b binding domain. Isolated endolysin proteins show broad-spectrum lytic activity especially against Gram-positive bacteria strains *Staphylococcus, Streptococcus, Enterococcus* and *Listeria* (Patent Number EP3252155A1).

The endolysin protein EN534-C contains two catalytic domains: amidase_3 and CHAP, and a LysM binding domain located in the central region of the molecule. Originally, the same sequence was found in the genome of the strain *Bacillus licheniformis* (Kuroda et al., 1992) and was described as a cell wall hydrolase gene and cloned. Identical sequences, designated N-acetylmuramoyl-L-alanine amidase (WP_017646700), were found in whole-genome sequence analysis of multiple strain genomes *Streptococcus agalactiae* for example *Streptococcus agalactiae* BSU454 (GenBank GCA_000311665.1). However, to date, the sequence of this gene for N-acetylmuramoyl-L-alanine amidase from the genomes of human isolates of *Streptococcus agalactiae* has not been cloned, and neither has the coding protein been overproduced or isolated.

The arrangement of catalytic domains amidase_3 and CHAP and the LysM binding domain has also been found in the endolysin PlySK1249 from the prophage of the strain *Streptococcus dysgalactiae subsp. equimilis*SK1249. However, at the amino acid level, the sequence homology of PlySK1249 and of endolysin EN534-C is only 75%.

PlySK1249 is the subject of document US20160145591A1.

**Summary of the invention**

EN534-C is the first endolysin derived from the human clinical isolate *Streptococcus agalactiae* KMB-534 (GBS) with an arrangement of the amidase_3 and CHAP and LysM domains that has been prepared in the pET28a+ vector as a recombinant protein. Based on methods for determining the lytic activity of recombinant endolysin EN534-C on a spectrum of substrates, its specific action against streptococcal strains, especially against the pathogenic bacteria *Streptococcus agalactiae* (GBS), was confirmed. The recombinant protein EN534-C has a pH optimum of 4.0 to 8.0. pH of 3.5-4.5 corresponding to the optimal pH in the vagina of healthy women, and an increasing pH indicates a vaginal bacterial infection.

The antimicrobial protein with lytic properties contains a sequence of the isolated protein according to SEQ ID NO. 1 or the recombinant protein sequence according to SEQ ID NO. 2.

According to a preferred embodiment, the antimicrobial recombinant protein has a pH optimum for lytic activity in the range of pH 4.0 to 8.0.

According to a preferred embodiment, the antimicrobial recombinant protein at a temperature in the range of 20°C to 40°C, and at a pH in the range of 5.0 to 8.0 has at least 60% lytic activity on human isolates of *Streptococcus agalactiae.*

According to a preferred embodiment, the antimicrobial recombinant protein has 60% to 97% lytic activity on human isolates of *Streptococcus agalactiae* or on the bacteria *Streptococcus agalactiae.*

The essence of the present invention consists also in the expression vector containing the sequence SEQ ID NO. 2 according to the present invention.

The recombinant antimicrobial protein is suitable for use in the treatment, prevention or diagnosis of diseases caused by the pathogenic bacteria *Streptococcus agalactiae* (GBS).

The essence of the present invention consists also in the process for preparation of the antimicrobial recombinant protein with lytic properties according to the present invention, its overproduction and isolation comprising the following steps:

a) preparation of the pET-EN534-CHis expression plasmid by cloning the amplified PCR fragment with the endolysin gene from prophage 3 from the strain *Streptococcus agalactiae* KMB-534 into the vector pET28a+;
b) transformation of the host cells *E. coli* BL21 (DE3) by the construct pET28-EN534-CHis;
c) growth of the cell culture at 37°C with constant shaking until it reaches an OD at 600 nm in the range of 0.5 to 0.6;
d) induction of cell culture with 0.4 mmol.l$^{-1}$ IPTG;
e) growth of the culture for at least 1 to 4 hours at a temperature of 28°C to 32°C with permanent shaking;
f) separation of bacterial cells from the medium;
g) resuspension of the cells in an equilibration solution;
h) adding a mixture of protease inhibitors in a volume of 10 $\mu$l/1 ml of suspension;
i) homogenisation of cells using sonication;
j) separation of the supernatant with the soluble protein fraction by centrifugation;
k) protein isolation by IMAC at laboratory temperature;
l) equilibrating the column, loading the fraction with protein, washing and eluting the protein.

The essence of the present invention consists also in an antimicrobial composition comprising an antimicrobial recombinant protein and a pharmaceutically acceptable carrier.

According to a preferred embodiment, an antimicrobial composition as well as an antimicrobial recombinant protein according to the invention are suitable for use as a medicament.

According to a preferred embodiment, the antimicrobial recombinant protein as well as the antimicrobial composition containing it are suitable for use in the treatment of multidrug-resistant streptococcal infections in pregnant women, neonates, urogenital infections, non-healing wounds and venous ulcers in adults, and mastitis and superficial infections in animals.

According to another preferred embodiment, the antimicrobial recombinant protein is suitable for decontaminating surfaces on which streptococci occur, comprising the step of contacting the surface with an effective amount of the antimicrobial protein, said protein having lytic activity against the bacteria *Streptococcus agalactiae* at a pH in the range of 4.0 to 8.0.

The present invention relates to the novel endolysin EN534-C derived from the tempered bacteriophage strain *Streptococcus agalactiae* KMB-534. The present invention relates to the preparation of a construct and method for overproduction of the recombinant protein EN534-C prepared in fusion with six histidine residues for its efficient isolation by affinity chromatography.

The isolated endolysin acts exolytically (from outside) on the cells of Gram-positive bacteria and is specific against streptococcal strains. The action of the endolysin removes pathogenic bacteria from the site of infection. The recombinant antimicrobial protein is suitable for use in the treatment, prevention and diagnosis of diseases caused by the pathogenic bacteria *Streptococcus agalactiae* (GBS). In the case of GBS infections caused by multidrug-resistant strains, recombinant endolysins can be administered synergistically with antibiotics to increase the effect.

Endolysin EN534-C was isolated from tempered bacteriophage 3 from the human isolate strain *Streptococcus agalactiae* KMB-534. The recombinant endolysin EN534-C was overproduced in sufficient quantities in the expression system *Escherichia coli.* Recombinant endolysin is isolated by affinity chromatography in soluble form, in sufficient quantity and activity. It has 90% relative lytic activity against streptococcal bacterial cells and is 75-60% active against other selected Gram-positive bacteria. It does not affect strains of *Lactobacillus.* It is active at pH from 4.0 - 8.0 and at temperatures from 20°C to 40°C.

The idea of the original determination of the use of the present invention is the application of recombinant endolysin especially in recurrent infections. Its application is also suitable for patients where the use of antibiotics is not recommended and thus will expand treatment options. The properties of the recombinant protein indicate that the phage endolysin is also suitable for use in veterinary medicine or in prophylaxis and disinfection in healthcare.

The novel EN534-C endolysin provides new possibilities for the diagnosis of GBS bacterial diseases, their prevention and/or treatment of streptococcal infection. It can be used in the case of multi-resistant streptococcal infections, not only in pregnant women and neonates, but also in adults.

The preparation or composition of EN534-C endolysin is also suitable in cases of vaginal bacteremia, urogenital infections, in applications and treatment of non-healing wounds and venous ulcers infected with streptococci.

EN534-C is also suitable for use in the veterinary field, e.g. for the treatment of mastitis or local infections in animals.

The isolated endolysin preparation or its composition can also be used for decontamination of surfaces in connection with the occurrence of streptococci and as a surface cleaning agent.

Detailed description

The use of phages and phage proteins as antimicrobial agents has several advantages over antibiotics. The main characteristic of phages and endolysins is their high specificity. Bacteriophages usually recognise and infect only a limited range of bacterial strains, which is an advantage in therapeutic use in terms of maintaining the host's normal microflora. The use of phage therapy appears to be very attractive in the treatment of urogenital tract infections in pregnant women, as it is possible to avoid dysbiosis and candidiasis, which are common side effects of antibiotics in the treatment of these infections. Endolysins also have specific lytic activity and are therefore considered to be antimicrobial agents suitable for treatment of infections caused by Gram-positive bacteria. Gram-positive bacteria do not have a cell wall outer membrane, and thus externally added endolysins have access to the peptidoglycan of the bacterial cell wall, allowing degradation and subsequent lysis and killing of the bacteria. The use of endolysins to treat bacterial infections also has advantages over therapeutic bacteriophages because it reduces the risk of developing resistance to therapeutic phages (Fischetti, 2010).

The invention relates to the preparation of a construct of the expression vector pET-EN534-CHis containing the nucleotide sequence of the endolysin gene (peg 1392) from prophage 3 of the human isolate strain *Streptococcus agalactiae* KMB-534, as a means to obtain the recombinant endolysin protein EN534-C with novel lytic properties. The recombinant protein contains a unique cluster of domains with lytic and binding activity in fusion with six histidines. Through the LysM binding domain, endolysin binds specifically to the peptidoglycan substrate ligand, followed by cleavage of the chemical bond in the peptidoglycan of the streptococcal bacterial cell wall by the enzymatic activity of the amidase_3 and CHAP lytic domains. The binding domain determines the range of spectrum of bacteria against which endolysin acts.

The invention also relates to the production of a recombinant peptide in *Escherichia coli* cells in soluble form. The invention also relates to the isolation procedure using IMAC affinity chromatography. The isolated recombinant endolysin is characterized by lytic activity against bacterial cells of pathogenic strains of *Streptococcus agalactiae* (GBS). Determining endolysin lytic activity is essential in diagnosing and testing the efficacy of recombinant endolysin against streptococci isolated from the site of infection.

**Brief description of the drawings**

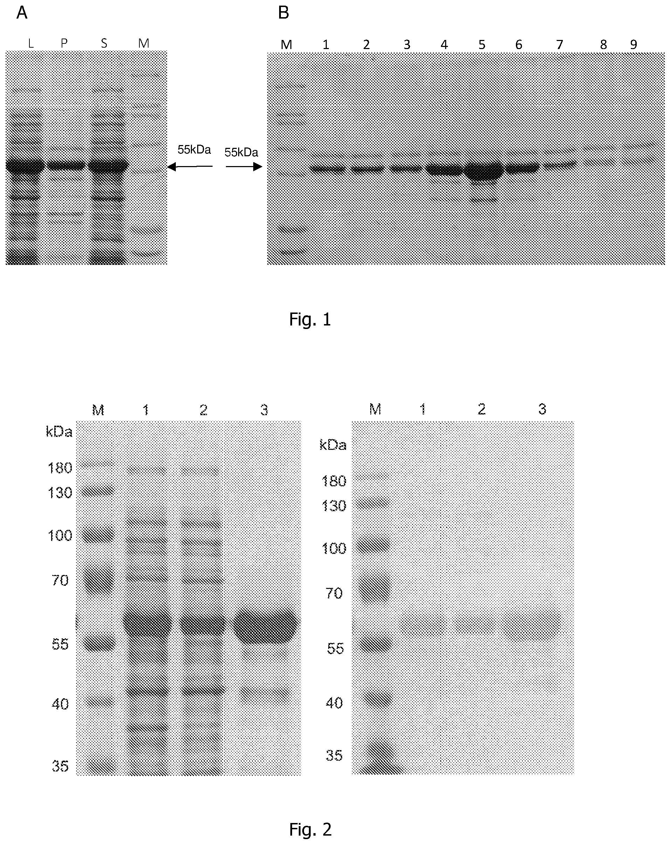

Fig. 1 - 10% SDS-PAGE expressions of the recombinant endolysin EN534-C in cells *E.coli BL21(DE3)* and the progression of isolation via a cobalt-bound chromatographic agarose support (Co-NTA).

A) expression of the recombinant protein EN534-C: L - lysate after cell sonication, P - cell pellet, S - supernatant, M - Mark12 protein standard (Thermo Fisher Scientific)
B) isolation via chromatographic agarose support with bound cobalt. Lanes 1.-2.-washing, 3.-9. elution fraction

M - Mark12 protein standard (Thermo Fisher Scientific)
Arrows point to expressed recombinant endolysin EN534-C of a size 55 kDa

Fig. 2 - 10% SDS-PAGE Detection of the C-terminal His-Tag anchor of the expressed recombinant protein EN534-C and Western Blot analysis was performed using a His-Tag monoclonal antibody.
M - Mark12 protein standard (26616, Thermo Fisher Scientific), 1. Induced culture lysate containing expressed recombinant protein EN534-C, 2. Soluble fraction of induced culture lysate with expressed recombinant EN534-C (Supernatant), 3. Isolated recombinant protein EN534-C using affinity chromatography

Fig. 3 - Lytic activity of recombinant endolysin EN534-C against bacterial cells of the strain *Streptococcus agalactiae* CCM 6187 measured spectrophotometrically.
Legend :

Solid square - control - *Streptococcus agalactiae* CCM 6187 in the absence of endolysin,
Solid triangle - substrate of *Streptococcus agalactiae* CCM 6187 after adding 0.1 $\mu$mol.ml$^{-1}$ of recombinant protein EN534-C.

Fig. 4 - Antimicrobial spectrum of recombinant protein EN534-C.
Optical density was measured on bacterial cell substrates of selected Gram-positive and Gram-negative strains after addition of the recombinant endolysin EN534-C (0.1 $\mu$mol.ml$^{-1}$). We determined the relative lytic activity (%) as the ratio of the decrease in optical density $OD_{600}$ of a control sample of the substrate (in the absence of recombinant endolysin) against a sample with recombinant endolysin over a measurement time of 60 minutes. The size of the error bars corresponds to the standard deviation (SD) calculated from three independently measured experiments.

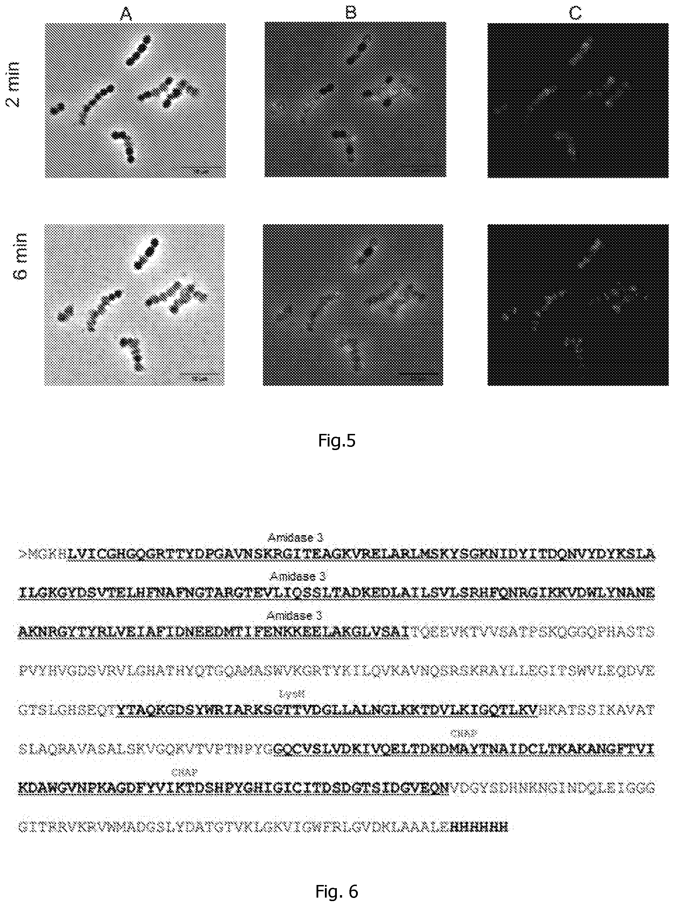

Fig. 5 - The monitoring of recombinant endolysin EN534-C lytic activity on a bacterial substrate *S. agalactiae* CCM 6187 in real time and at minute intervals.
Processed bacterial cells *S. agalactiae* CCM 6187. Figures (A) greyscale - phase contrast image and (B) fluorescence image (SYTOX green), show bacterial cells after addition of endolysin - black spheres are live cells, light grey - cells after lysis (peptidoglycan lysis has occurred), SYTOX green - green colour shows the eluted DNA content, which is shown in Figure 5B by a light grey zone around the bacterial cells (peptidoglycan lysis) and the visible eluted DNA content of the cells after endolysin addition, (C) fluorescence image of cells stained with FM 4-64 - membrane structures visibly marked in living cells only (before and after the addition of endolysin). The scale corresponds to 10 $\mu$m.

Fig. 6 - Amino acid sequence of recombinant endolysin EN534-C
Domain sequences in AK are underlined and bold: **Amidase 3:** L5 - 1169; binding domain **LysM:** Y270 - V312; **CHAP:** G351 - N433; **His-Tag:** H499 - H504

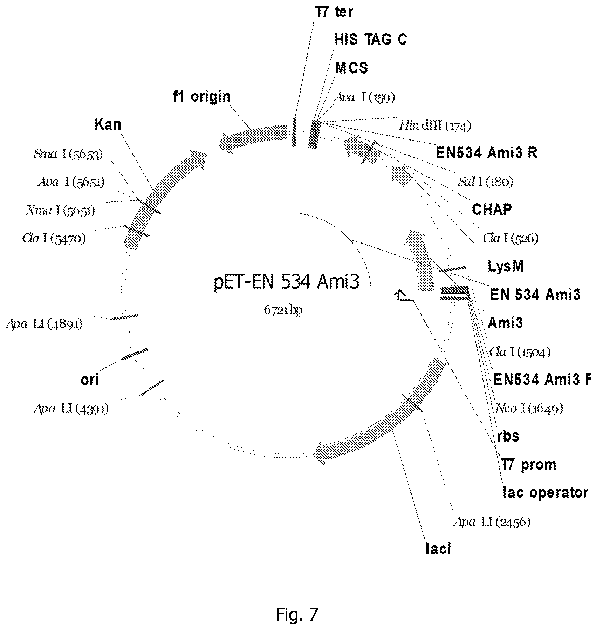

Fig. 7 - Map of the pET28-EN534-CHis vector. The construct was prepared by cloning an amplified PCR fragment with the EN534 endolysin gene (peg 1392) from prophage 3 from the strain *Streptococcus agalactiae* KMB-534 into the vector pET28a+. Recombinant endolysin is in fusion with the C-terminal His-Tag.
Cloning sites for endonucleases *Nco*I and *Sal*I, sites for the forward (EN534 Ami3 F) and reverse (EN534 Ami3 R) primers, and fragment with the endolysin sequence are indicated: EN534 Ami3 and lytic domain sequences: Ami3 (amidase 3) and CHAP and the LysM binding domain.

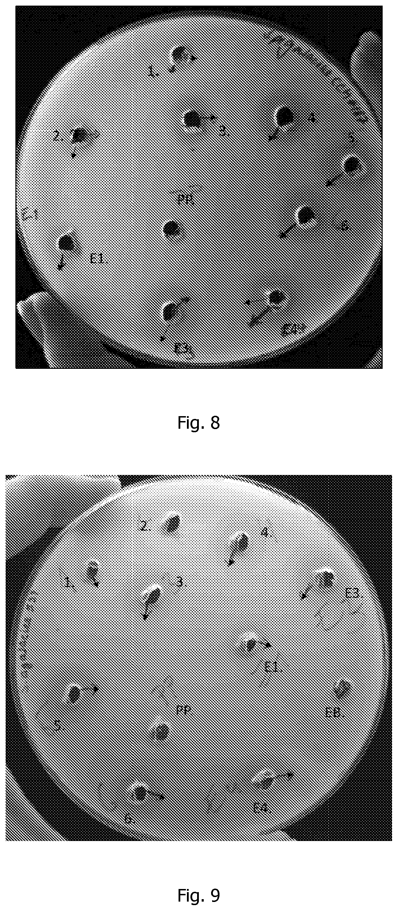

Fig. 8 - Example of lytic activity determination of endolysin EN534-C by diffusion method on a substrate of *S. agalactiae* CCM 6187 cell walls.

Fig. 9 - Example of lytic activity determination of endolysin EN534-C by diffusion method on a substrate of *S. agalactiae* KMB-534 cell walls.

**Examples of embodiment**

Work with microorganisms

We stored the bacterial strains *Escherichia coli, Bacillus subtilis, Enterococcus faecalis, Lactobacillus jensenii* and *Streptococcus agalactiae* in 40% (v/v) glycerol at -80°C. We cultured the bacterial strains in various solid and liquid media: LB medium *(Escherichia coli, Bacillus subtilis),* THB medium *(Streptococcus agalactiae),* MRS medium *(Lactobacillus jensenii)* and BHI medium *(Enterococcus faecalis).* The cultivation temperature was 37°C for *Escherichia coli, Streptococcus agalactiae, Lactobacillus jensenii, Enterococcus faecalis,* and 30°C for *Bacillus subtilis.* Cultivation in liquid media was performed with permanent shaking on an I26R rotary shaker (New Brunswick Scientific, USA) at 200 rpm$^{-1}$; in the case of *Lactobacillus jensenii,* cultivation was performed under anaerobic conditions. When culturing bacteria containing plasmids carrying the kanamycin resistance gene, kanamycin was added to the culture medium to - a final concentration of 100 $\mu$g.ml$^{-1}$.

Table 1. Used bacterial strains and endolysin

| Bacterial strains/ | Genotype/characteristics | Reference |
|---|---|---|
| *Streptococcus agalactiae* CCM 6187 | *Streptococcus agalactiae* Leh mann and Neumann 1896$^{AL}$/Lancefield's group B, non-haemolytic | Czech Collection of Microorganisms (CCM), Brno, Czech Republic |
| *Bacillus subtilis* wt PY79 | prototrophic derivative *B. subtilis* 168 | Institute of Molecular Biology, Slovak Academy of Sciences, Slovak Republic |
| *Escherichia coli* BL21(DE3) | F$^-$, ompT, hsdS$_B$ (r$_B^-$ m$_B^-$), gal, dcm (DE3) | Novagen, USA |
| *Escherichia coli* XL-1 Blue | endA1 gyrA96(nal$^R$) thi-1 recA1 relA1 lac glnV44 F'[::Tn10 proAB$^+$ lacI$^q$ $\Delta$(lacZ)M15] hsdR17(r$_K^-$ m$_K^+$) | Stratagene, USA |
| *Escherichia coli* NiCo21(DE3) | can::CBD fhuA2 [lon] ompT gal ($\lambda$ DE3) [dcm] arnA::CBD slyD::CBD glmS6Ala $\Delta$hsdS $\lambda$ DE3 = $\lambda$ sBamHIo $\Delta$EcoRI-B int:: (lacI:: PlacUV5:: T7 gene1) i21 $\Delta$nin5 | NEB, USA |
| *Streptococcus agalactiae* KMB-534 | Serotype III, MLST ST-17, Rib surface protein | human urine isolate, Department of Molecular Biology, Faculty of Natural Sciences Comenius University Bratislava, Slovak Republic |
| *Streptococcus agalactiae* KMB-533 | Serotype V, MLST ST-1, Rib surface Alp 2/3 | human urine isolate, Department of Molecular Biology, Faculty of Natural Sciences Comenius University Bratislava, Slovak Republic |
| *Streptococcus agalactiae* KMB-548 | Serotype VII, MLST ST-19, Rib surface protein | human urine isolate, Department of Molecular Biology, Faculty of Natural Sciences Comenius University Bratislava, Slovak Republic |
| *Streptococcus agalactiae* KMB-572 | Serotype III, MLST ST-130, Alpha C surface protein | human isolate from vaginal swab, Department of Molecular Biology, Faculty of Natural Sciences Comenius University Bratislava, Slovak Republic |
| *Lactobacillus jensenii* | | human vaginal swab isolate, Department of Cellular and Molecular Biology of Drugs, Faculty of Pharmacy Comenius University Bratislava, Slovak Republic |
| Endolyzín EN534 | Peg 1392, prophage 3 (group B) strain *Streptococcus agalactiae* KMB-534 | subject of the patent |

Table 2. Used culture media

| Culture medium | Media composition / Manufacturer |
|---|---|
| 1x LB medium (liquid) | 10 g.l$^{-1}$ trypton (Duchefa Biochemistry, Netherlands) |
| | 5 g.l$^{-1}$ yeast autolysate (Sigma-Aldrich, USA) |
| | 10 g.l$^{-1}$ NaCl (Centralchem, Slovakia) |
| | pH 7.0 |
| LB medium (solid) | LB medium |
| | 15 g.l$^{-1}$ agar (MP Biomedicals, France) |
| TB medium (liquid) | 12 g.l$^{-1}$ trypton (Duchefa Biochemie, Netherlands) |
| | 24 g.l$^{-1}$ yeast autolysate (Sigma-Aldrich, USA) |
| | 8 ml glycerol (Centralchem, Slovakia) |
| | 9.4 g.l$^{-1}$ K$_2$HPO$_4$ (Slavus, Slovakia) |
| | 2.2 g.l$^{-1}$ KH$_2$PO$_4$ (Slavus, Slovakia) |
| 1x THB medium (liquid) | 36.4 g.l$^{-1}$ THB (Biolife, Italy) pH 7.8 |
| THB medium (solid) | 36.4 g.l$^{-1}$ THB (Biolife, Italy) |
| | 15 g.l$^{-1}$ agar (MP Biomedicals, France) |
| 1x BHI medium (liquid) | 37 g.l$^{-1}$ BHI (Oxoid, UK) |
| | pH 7.4 |
| BHI medium (solid) | 37 g.l$^{-1}$ BHI (Oxoid, UK) |
| | 15 g.l$^{-1}$ agar (MP Biomedicals, France) |
| 1x MRS medium (liquid) | 51 g.l$^{-1}$ MRS (Sigma-Aldrich, USA) |
| | 1 ml.l$^{-1}$ Tween 80 (Amresco, USA) |
| | pH 6.2 |
| MRS medium (solid) | 51 g.l$^{-1}$ MRS (Sigma-Aldrich, USA) |
| | 1 ml.l$^{-1}$ Tween 80 (Amresco, USA) |
| | 15 g.l$^{-1}$ agar (MP Biomedicals, France) |
| 1x Mueller-Hinton Broth (MHB) medium (liquid) | 21 g.l$^{-1}$ MHB (Sigma-Aldrich, USA) |
| | pH 7.4 |
| Mueller-Hinton Broth medium (solid) | 21 g.l$^{-1}$ MHB (Sigma-Aldrich, USA) |
| | 15 g.l$^{-1}$ agar (MP Biomedicals, France) |

DNA manipulation

Preparation of the expression plasmid pET-EN534-CHis by cloning an amplified PCR fragment with the EN534 endolysin gene from prophage 3 of the strain *Streptococcus agalactiae* KMB-534 into the vector pET28a+

Isolation of chromosomal DNA from streptococci

To isolate chromosomal DNA from the strain *Streptococcus agalactiae* KMB-534 we used the DNeasy Blood & Tissue Kit (Qiagen, Germany). We followed the protocol recommended by the manufacturer.

Amplification of DNA fragments by PCR, cleavage by restriction endonucleases and isolation of DNA fragments from an agarose gel

The specific primers listed in Table 3 were used in the PCR reaction to amplify DNA fragments with the endolysin gene. Chromosomal DNA isolated from the bacterial cells of the strain *Streptococcus agalactiae* KMB-534 was used as a template for PCR amplification of the respective fragment.

Table 3. Primers used

| Primers | Sequence* | $T_a$ (in PCR) ** |
|---|---|---|
| EN 534_Ami3 F | 5'- ATACCATGGGAAAACATCTAGTGA-3' | 65°C |
| EN 534_Ami3 R | 5'-CTTGTCGACTCCTAATCTGAACCAA -3' | |

\* Restriction endonuclease sites *NcoI and SaiI are* underlined

\*\* $T_a$ is the annealing temperature in the PCR reaction

The individual components of the PCR reaction and their concentrations are listed in Table 4. The temperature cycle of the PCR reactions is shown in Table 5. DNA fragment amplification proceeded in multiple parallel reactions in a T-gradient thermocycler (Whatman Biometra, Germany). The quantity and quality of the PCR products were analysed in a 1% agarose gel electrophoresis. After combining several identical reaction mixtures, the PCR product was purified using a "QIAquick PCR Purification Kit" (Qiagen, Germany) and subsequently cleaved using the respective restriction endonucleases *NcoI* and *Sal*I (NEB, USA). After fragment separation on preparative 0.8% agarose electrophoresis, the corresponding DNA fragment was isolated from the gel using the "QIAquick Gel Extraction Kit" (Qiagen, Germany). The purified PCR fragment was further used in the ligation reaction together with the linearised vector pET28a+.

Table 4. Composition of the PCR reaction mixture

| PCR components | Final concentration |
|---|---|
| HF buffer solution for Phusion High-fidelity DNA | 1 x concentrated |
| dNTP (composed of dATP, dCTP, dGTP, dTTP) | 0.2 mmol.l$^{-1}$ |
| Forward primer | 0.5 $\mu$mol.l$^{-1}$ |
| Reverse primer | 0.5 $\mu$mol.l$^{-1}$ |
| MgCl$_2$ | 1.5 - 2 mmol.l$^{-1}$ |
| template DNA | 100 ng |
| Phusion High-fidelity DNA polymerase | 1U |

The reaction mixture was made up to a final volume of 50 $\mu$l with sterile redistilled water. In the case of the negative control, we replaced the DNA with sterile redistilled water.

Table 5. Temperature program for PCR amplification

| Number | Amplification phases | emperature | Time |
|---|---|---|---|
| 1 | Initial denaturation | 98°C | 5 min |
| 35 | Denaturation | 98°C | 30 s |
| | Annealing | 65°C | 20 s |
| | Polymerisation | 72°C | 60 s |
| 1 | Final polymerisation | 72°C | 10 min |
| | Cooling | 4°C | ∞ |

Isolation of plasmid DNA from *Escherichia coli*

Various methods were used to isolate plasmid DNA. The boiling method was used to isolate analytical amounts of plasmid DNA for rapid analysis of transformants. The "Qiagen Plasmid Kit" (Qiagen, Germany) was used to isolate larger amounts of plasmid DNA for DNA sequencing or manipulation.

Cleavage of plasmid DNA by restriction endonucleases and dephosphorylation

Plasmid DNA pET28a+ was cleaved using restriction endonucleases *Nco*I and *Sal*I in a reaction mixture under the conditions recommended by the manufacturer (NEB, USA). After cleavage with restriction endonucleases, we treated the pET28a+ plasmid DNA with alkaline phosphatase under the conditions recommended by the manufacturer (NEB, USA). The dephosphorylated linearised plasmid DNA was separated on a preparative agarose gel and subsequently isolated from the gel using the "QIAquick Gel Extraction Kit" (Qiagen, Germany). Plasmid DNA concentration was determined spectrophotometrically on a NanoDrop 2000c instrument (Thermo Scientific, Germany) or fluorometrically on a Qubit instrument using the "Quant-iT™ dsDNA BR Assay Kit" (Invitrogen, USA). The plasmid thus prepared was used in the ligation reaction.

Ligation and transformation

T4 DNA ligase was used to ligate the linearised vector and PCR fragments under the reaction conditions recommended by the manufacturer (NEB, USA). ATP was added to the reaction to a final concentration of 2 mmol.l$^{-1}$. The relative molar ratio of the vector and insert in the ligation mixture was 1:3, wherein the amount of the vector was 100 - 200 ng. We transformed the *Escherichia coli* XL-1 Blue competent cells with the ligation mixture. The transformed cells were spread on solid LB medium with added kanamycin and allowed to grow in a thermostat at 37°C for 16 - 18 hours. Plasmid DNA isolated from transformants was analysed by restriction endonuclease cleavage.

DNA electrophoresis in agarose gels

DNA was analysed in 0.8 - 1% agarose gels by horizontal electrophoresis. Analytical electrophoresis was prepared in a BBE buffer solution, and preparative electrophoresis in a TAE solution. After electrophoresis, DNA fragments were visualised using a UV lamp (Transiluminator Vilber Lourmat, France) at the wavelength 254 nm (analytical) or 365 nm (preparative electrophoresis). DNA Marker, 3-Lambda DNA/EcoRI+HindIII (Thermo Fisher Scientific, USA) or GeneRuler 100 bp DNA Ladder Plus (Thermo Fisher Scientific, USA) were used as the molecular size standard.

Table 6. Prepared and used plasmids

| Plasmid | Genotype! characteristics | reference |
|---|---|---|
| pET28a+ | 5369 bp Km$^r$ *f1*(+) ori oriC from the vector pBR322 His$_6$tag, T7 promoter with lacUV operator, lacI, host: *Escherichia coli* | Novagen, USA |
| pET-EN534-CHis | pET28a+ derivative containing a fragment with the endolysin gene from prophage 3 (peg 1392) from *S. agalactiae* | Subject of the patent |

Overproduction in *Escherichia coli* cells and isolation of the EN534-C protein by IMAC

Induction of protein expression in EN534-C

We used host strains of *Escherichia coli* BL21(DE3) (Novagen, USA) and NiCo21(DE3) (NEB, USA) to overproduce the EN534-C protein. We transformed *Escherichia coli* cells with the prepared plasmid construct, and selected individual colonies grown after transformation were inoculated into 3 ml of liquid LB medium with added kanamycin (100 $\mu$g.ml$^{-1}$). The culture was grown overnight on a I26R rotary shaker at 200 rpm$^{-1}$ and temperature of 37°C. From the grown overnight culture, we inoculated 1 ml into 100 ml of TB medium with added kanamycin (100 $\mu$g.ml$^{-1}$). The cell culture was grown on a rotary shaker at 37°C until the absorbance at wavelength 600 nm reached 0.5 - 0.6. The culture was cooled to 30°C and protein expression was induced by adding the inducer IPTG (isopropyl-$\beta$-D-1-thiogalactopyranoside) to a final concentration of 0.4 mmol.l$^{-1}$. The culture was cultivated for further 2 hours at 30°C with permanent shaking. Subsequently, the cells were sedimented on an Eppendorf 5810R centrifuge (15 min, 6000 rpm$^{-1}$, 4°C), the cell sediment was washed with saline (0.9% (w/v) NaCl) and centrifuged again (15 min, 6000 rpm$^{-1}$, 4°C). The cell sediment was frozen with liquid nitrogen and stored at -20°C or -80°C, if applicable.

Preparation of cell lysate

The cells were thawed on ice, WEQ equilibration solution (Table 7) was added in a ratio of 1 ml per 20 ml in volume of the original culture medium and resuspended. Subsequently, a mixture of protease inhibitors containing AEBSF, phosphoramidone, peptidase A, bestatin and E-64 (Protease Inhibitor Cocktail, Sigma-Aldrich, USA) was added in a volume of 10 $\mu$l/1 ml of suspension. The cell suspension was homogenized using sonication on a Soniprep 150 plus (MSE) at an amplitude of 10, for a period of 15 seconds with a cooling break of 2 minutes. We repeated the intervals a maximum of 5 times. The sample was cooled on ice throughout the sonication process. The cell lysate was centrifuged for 30 minutes at 4°C and 14 000 rpm$^{-1}$ (Eppendorf 5417R). We separated the insoluble part (pellet, sediment) and the soluble fraction (supernatant) by centrifugation. We verified the presence of recombinant protein in the fraction using denaturing protein acrylamide gel electrophoresis (SDS-PAGE) and visualised it by staining.

Table 7. Used solutions and their composition

| | Solutions | Composition |
|---|---|---|
| **Determination of lytic activity** | **Solution A** – reaction solution | 50 mmol.l$^{-1}$ Tris-HCl pH 8.0; 10 mmol.l$^{-1}$ NaCl, 10 mmol.l$^{-1}$ CaCl$_2$ |
| | **SM buffer** solution | 50 mmol.l$^{-1}$ Tris-HCl pH 7.5; 10 mmol.l$^{-1}$ NaCl, 8 mmol.l$^{-1}$ MgSO$_4$x7H$_2$O |
| | **TN solution** – washing solution | 50 mmol.l$^{-1}$ Tris-HCl pH 8.0; 0.9% NaCl, 1 % glycerol |
| **Protein isolation** | **WEQ solution**: washing, sonication and equilibration solution for EN534-C isolation | 50 mmol.l$^{-1}$ Tris-HCl pH 8.0; 500 mmol.l$^{-1}$ NaCl, 10 mmol.l$^{-1}$ imidazol |
| | **Solution E**: Elution solution | 50 mmol.l$^{-1}$ Tris-HCl pH 8.0; 500 mmol.l$^{-1}$ NaCl, 500 mmol.l$^{-1}$ imidazol |
| | Dialysis solution D | 50 mmol.l$^{-1}$ Tris-HCl pH 8.0; 150 mmol.l$^{-1}$ NaCl, 10 % (v/v) glycerol |
| | Saline solution | 0.9 % NaCl (w/v) |
| **Western Blot** | Transfer buffer solution | 25 mmol.l$^{-1}$ Tris-HCl pH 8.3; 0.192 mol.l$^{-1}$ NaCl, 20 % (v/v) methanol |
| | 10 x TBS | 100 mmol.l$^{-1}$ Tris-HCl pH 7.5; 1.5 mol.l$^{-1}$ NaCl |
| | TTBS | 1x TBS, Tween 20 (35 µl/100 ml of solution) |
| | Blocking solution | 300 mg BSA, 10 ml 1x TBS |

| | | |
|---|---|---|
| | Antibody solution | 100 mg BSA, 10 ml 1x TBS |
| | Detection solution | 100 mmol.l⁻¹ Tris-HCl pH 9.5; 100 mmol.l⁻¹ NaCl |
| | Developing solution | 150 µl BCIP/NBT, 7.35 ml detection solution |
| **SDS-PAGE proteins** | 10x TGS solution (PAGE-SDS) | 25 mmol.l⁻¹ Tris-HCl pH 8.3; 200 mmol.l⁻¹ glycine; 1 g.l⁻¹ SDS |
| | Staining solution | 25% (v/v) isopropyl alcohol, 10% (v/v) acetic acid, 1 gl⁻¹ CBB R-250 (Comassie Brilliant Blue) |
| | Destaining solution | 10% (v/v) acetic acid, 10% (v/v) methanol |
| | Fixing solution | 50% (v/v) methanol, 10% (v/v) acetic acid |
| **DNA electrophores** | 10 x BBE solution | 3 mmol.l⁻¹ sodium tetraborate pH 7.8; 65 mmol.l⁻¹ boric acid, 2.5 mmol.l⁻¹ EDTA |
| | 10 x TAE solution | 30 mmol.l⁻¹ Tris-HCl pH 8.0; 20 mmol.l⁻¹ acetic acid, 1 mmol.l⁻¹ EDTA |

Isolation of the EN534-C protein by IMAC

The soluble fraction containing the recombinant protein was filtered through a bacteriological filter (0.45 µm, Millipore, USA). The recombinant protein was isolated by metal chelate affinity chromatography (IMAC). We used a chromatographic agarose support with bound cobalt or nickel (His-Select cobalt affinity gel; His-Select nickel affinity gel, Sigma-Aldrich, USA) in the column. Protein isolation was carried out at laboratory temperature. The column was washed with redistilled water and equilibrated with WEQ wash solution before applying the soluble fraction (Table 7). Subsequently, we applied the protein fraction. After such application, non-specifically bound proteins were removed by washing the support with WEQ wash solution in a volume of 7 times the column volume. The protein was eluted with elution buffer solution E (Table 7) in a volume of 3 times the column volume, and we collected the fractions of 500 µl. The protein content in the individual fractions was analysed by SDS-PAGE. Protein concentrations in the samples were determined fluorometrically using the Qubit Protein Assay Kit on a Qubit® 2.0 Fluorometer (Invitrogen, USA), spectrophotometrically on a NanoDrop Lite Spectrophotometer (Thermo Fisher Scientific, USA) and using Bradford analysis (Sigma-Aldrich, USA). We used an Amicon® Ultra-4 Centrifugal Filter Unit (Merck, USA) to exchange elution solution E for solution D (Table 7) in the resulting protein fractions and also we concentrated the isolated protein sample to the optimal concentration. The resulting EN534-C protein samples were frozen in nitrogen and stored at -20°C, or -80°C, if applicable.

In the case of the EN534-C recombinant protein overproduced in the *E. coli* NiCo21 (DE3) expression strain (NEB, USA), after sonication, centrifugation, and filtration through a 0.45 µm bacteriological filter, we applied the soluble fraction first on the column containing a chitin support (NEB, USA). The protein was incubated at 4°C with chitin support for 15 min and then the column was washed with solution of 50 mmol.l⁻¹ Tris-HCl pH 8.0; 500 mmol.l⁻¹ NaCl, at a flow rate of 0.5 ml.min⁻¹ and the overflow target recombinant protein was collected. The protein sample was further applied on a cobalt or nickel agarose support and the protein was subsequently isolated according to the previous procedure.

Denaturing electrophoresis of proteins in polyacrylamide gels

Protein denaturing electrophoresis of samples from protein expression and of individual fractions from the protein isolation were performed on 10% SDS-PAGE gels in a P8DS-1 apparatus (Thermo Fisher Scientific - Owl, USA). We used the Mark12 Unstained Standard (Invitrogen, USA) as a molecular weight standard. Electrophoresis was performed at a potential gradient of 7 - 10 V.cm$^{-1}$. The SDS-PAGE gel was stained for 30 min in the staining solution and de-stained for 2 x 30 min in the destaining solution (Table 7). Both staining and destaining of PAGE gels were performed at room temperature with gentle shaking.

Protein Immunodetection - Western Blot Analysis

Samples of recombinant protein containing the His-Tag anchor were separated on 10% SDS-PAGE, and the PageRuler Prestained Molecular Ladder 26616 (Thermo Fisher Scientific, USA) was used as a molecular weight standard. The separated proteins were electrophoretically transferred from the gel to a nitrocellulose membrane (Advantec, Japan) in a Panther™ semidry electroblotter Owl apparatus (Merck, USA). We performed the transfer in transfer buffer solution (Table 7) for 90 - 120 min at a current of 0.8 mA per 1 cm$^2$ of membrane. Next, we followed the TB283 protocol for colorimetric detection (Novagen, USA). The transferred protein membrane was incubated overnight (16 h) in blocking buffer solution (Table 7) at 4°C. The next day, we washed the membrane with the TTBS solution (Table 7) and incubated for 2 hours with a primary monoclonal antibody against the His-Tag sequence isolated from mice (His-Tag monoclonal antibody, Novagen). We diluted the primary antibody in antibody solution (Table 7) in a 1: 1000 ratio. Subsequently, we washed the membrane with the TTBS solution (Table 7) for 30 min and incubated for 1 hour with an alkaline phosphatase-conjugated secondary antibody (Goat Anti-Mouse IgG Alkaline Phosphatase Conjugate, Novagen). We diluted the secondary antibody in a 1:10 000 ratio. The membrane was washed twice for 10 min with the TTBS solution. The whole process was performed at laboratory temperature. Immunopositive proteins were visualised by colorimetric reaction using BCIP/NBT substrate detection solution (Roche Applied Science, Switzerland) (Table 7). The molecular weight of the immunodetected proteins was verified using the PageRulerTM Prestained Protein Ladder standard (Thermo Fisher Scientific, USA).

**Example 1** - Bioinformatics and sequencing analysis

For whole-genome sequencing of isolated strains *Streptococcus agalactiae* a Nextera XT DNA Library Prep Kit (Illumina) and the Illumina MiSeq sequencer were used at the Department of Molecular Biology, Faculty of Science, Comenius University, Bratislava. The region of the genome containing prophage sequences was detected using PHAST. The annotations obtained were filtered in the Rast program using the "subcategories" filter and based on the presence of the keyword "phage" in the annotation. The most suitable prophage sequences from whole-genome sequencing were selected and processed using the Vector NTI program. Potential endolysins in whole-genome sequences were identified using the phiBIOTICS database. The selected gene encoding endolysin from the prophage was analysed in the Blast database using BLASTX and BLASTP compared to a non-redundant protein database. The preparation of recombinant endolysin constructs was processed using the Vector NTI program. Below is a list of the software used for bioinformatics data analysis.
List of software used for bioinformatics data analysis:

BLAST - algorithm for searching for homologous sequences in the NCBI database (https://blast.ncbi.nlm.nih.ciov/Blast.ccii)
CLC Bio - bioinformatics software for analysis of data obtained from next-gen sequencers http://www.clcbio.com (Qiagen)
PubMLST - an international database of bacterial genomes to determine the diversity of microbial genomes (https://pubmlst.org/)
RAST/SEED - online program for annotation of genome sequences (http://rast.nmpdr.org/)
Vector NTI - program for analysis of nuclear and protein sequences (Invitrogen)
phiBIOTICS - online database for searching for potential bacteriophages and enzybiotics in whole-genome sequences (http://www.phibiotics.org/index.php)
PHAST - online server designed for identification, annotation and graphical display of prophage sequences in bacterial genomes (http://phast.wishartlab.com/)
PATRIC - program for comparison of bacterial genomes (https://www.patricbrc.org/) ImageJ Fiji - image processing program

Evaluation:

Gene for endolysin EN534 (peg. 1392) of size 1467 bp, is derived from prophage 3 (group B phage) (Table 8) from the genome of a human isolate strain *Streptococcus agalactiae* KMB-534 (Department of Molecular Biology, Faculty of Sciences, Comenius University). The endolysin protein is 489 AK in size. Using BLASTX and BLASTP (in the BLAST database), sequences with amidase_3, LysM, and CHAP functional domains were identified on the EN534 endolysin protein (Table 9).

Table 8. Basic characteristics of prophages in the whole genome sequence *Streptococcus agalactiae* KMB-534

| Strain | Prophage | Phage group | Contig* | Position of prophage sequence in contig** | Size (bp) | Peg*** | Number of genes |
|---|---|---|---|---|---|---|---|
| *Streptococcus agalactiae* KMB-534 | 1 | C | KMB-534_contig_4 | 62683-80605 | 17922 | 1504-1530 | 27 |
| | 2 | D | KMB-534_contig_10 | 66273-84358 | 18085 | 88-105 | 18 |
| | 3 | B | KMB-534_contig_32 | 3122-35347 | 32225 | 1359-1392 | 34 |

*Contig (contiguous) is a region of overlapping DNA sequences that together represent a consensus region

** The position in the contig corresponds to the section with the sequence of prophages (1, 2 and 3)

*** peg (protein encoding gene) – represents the sequence encoding the putative protein in a given contig from the prophage region in the whole-genome sequence

Table 9. Arrangement of functional domains on the endolysin protein EN534 (peg.1392) from the prophage 3 of the strain *Streptococcus agalactiae* KMB-534

| Domain | Domain location | Pfam domain | E-value | Domain function |
|---|---|---|---|---|
| MurNAc-LAA superfamily | 5-169 | Pfam01520 | 2.08e-13 | N-acetylmuramoyl-L-alanine amidase (MurNAc-LAA) - hydrolyzes the bond between N-acetylmuramic acid and L-alanine in peptidoglycan |
| LysM superfamily | 270-312 | Pfam01476 | 2.82e-10 | LysM binding domain - peptidoglycan binding domain |

(continued)

| Domain | Domain location | Pfam domain | E-value | Domain function |
|---|---|---|---|---|
| CHAP | 351-433 | Pfam05257 | 1.38e-07 | CHAP - hydrolysis of peptidoglycan bonds and bridges |

**Example 2** - Preparation of recombinant endolysin EN534-C

Preparation of expression plasmid pET-EN534-CHis containing the gene for recombinant endolysin EN534-C

The pET-EN534-CHis construct (Figure 7) was prepared using the expression vector pET28a+ (Novagen, USA). In addition to the T7 promoter for T7 RNA polymerase, the vector carries a His-Tag anchor coding sequence (a potential AK sequence with six histidines). The resulting recombinant protein may be fused to six histidines (His-Tag) at the N- and/or C-terminus. The presence of the His-Tag anchor and its localization on the protein may affect the expression of the fusion protein, its solubility, and the enzymatic activity of the protein. At the same time, His-Tag allows the isolation of the recombinant protein by affinity chromatography on a metal chelate support.

We chose the strategy of preparing recombinant protein EN534-C in fusion with six histidines at the C-terminus. For the preparation of the PCR fragment with the endolysin gene to be cloned, we designed specific primers - forward: EN 534_Ami3 F, and reverse: EN 534_Ami3 R (Table 3). The amplified fragment with the *EN534* endolysin gene sequence was cloned into the expression vector pET28a+ via the cloning sites *Nco*I a *Sal*I. The correctness of the nucleotide sequence of the cloned gene encoding recombinant endolysin in the prepared plasmid construct pET-EN534-CHis was verified by sequencing (GATC Biotech, Germany) and sequence analysis (Vector NTI).

Expression and isolation of recombinant protein EN534-C

The resulting recombinant protein EN534-C contains 504 amino acids and has a molecular weight $M_h$ of 55.02 kDa, pI 9.02 and contains the His-Tag sequence at the C-terminus of the protein (Table 9 and Figure 1).

We expressed the recombinant protein EN534-C from plasmid pET28-EN534-CHis in the expression strain *Escherichia* coli BL21(DE3) or NiCo21(DE3). We optimized the expression conditions - induction with 0.4 mmol.l$^{-1}$ IPTG at OD ~ 0.5, 2 hours at 30°C. The expressed protein was present in the soluble fraction (supernatant) in sufficient quantity for isolation, but at the same time part of the protein was also expressed in the insoluble fraction (sediment, pellet) (Figure 1A). The filtered supernatant was applied to a nickel-bound (Ni-NTA) or cobalt-bound (Co-NTA) chromatographic agarose support and isolated as described (see Isolation of EN534-C protein by IMAC). The protein isolation process and individual fractions are shown in Figure 1B. The size of the isolated recombinant protein EN534-C was confirmed by immunodetection with His-Tag monoclonal antibody using Western Blot analysis (Figure 2).

**Example 3** - Determination of enzymatic (lytic) activity of recombinant protein EN534-C by diffusion method

In the diffusion method, we used bacterial strains of *Streptococcus agalactiae* as a substrate, from which we prepared the cell wall substrate. Bacterial cultures were grown in THB medium on a I26R rotary shaker at 37°C and 200 rpm$^{-1}$ to the late phase of exponential growth ($OD_{600}$ ~ 1). The cells were sedimented by centrifugation on an Eppendorf 5810R centrifuge (4000 rpm$^{-1}$, 10 min, 4°C). The sediment was then resuspended in sterile water in a volume of 1/10 of the original culture volume, and the mixture was autoclaved for 15 minutes. Next, the cell suspension was centrifuged, the sediment was washed twice with sterile water (1/10 of the original culture volume), centrifuged again, and prepared the cell substrate was resuspended in sterile water to a final concentration of 20% (w/v). The cell substrate was used immediately or frozen in liquid nitrogen and stored at -80°C.

To determine the lytic activity of endolysin EN534-C by the diffusion method, we added to 0.6% (w/v) agarose boiled in solution: 50 mmol.l$^{-1}$ Tris-HCl pH 8.0, cell substrate to a final concentration of 1% (w/v), mixed and poured into sterile Petri dishes. After solidification, we cut wells into the agarose, to which we applied 10 $\mu$mol.l$^{-1}$ of the isolated protein solution in a volume of 10 $\mu$l. Plates with applied samples were incubated overnight in a thermostat at 37 °C. We identified lytic activity in the form of a clear zone around the applied endolysin.

Evaluation:

The recombinant protein EN534-C contains three enzymatic domains, two of which are catalytic (N-terminal amidase_3, C-terminal CHAP) and one binding (LysM - in the central part) (Table 9 and Figure 6). The lytic activity of endolysin EN534-C is the result of degradation of the bacterial cell wall substrate by the action of two catalytic domains. Lytic activity was determined using the diffusion method on cell wall substrates of *S. agalactiae* CCM 6187 and *S. agalactiae* KMB-534. Samples with recombinant protein EN534-C were applied to wells on an agarose plate containing cell wall substrates. We used WEQ equilibration solution in a volume of 10 μl as a negative control. Lytic activity in the form of a clear zone was detected after 16 h for all samples that contained the recombinant protein EN534-C. On a substrate plate of *S. agalactiae* CCM 6187 (Figure 8) and *S. agalactiae* KMB-534 (Figure 9) there are seen clear zones in samples with *E.coli* culture lysate after induction - containing the expressed recombinant protein EN534-C (samples 1-6), and also in a sample with isolated recombinant protein EN534-C (samples E1-E4).

The resulting lytic activity corresponds to the width of the lytic zone $\mathbf{h} = (d_{\text{lytic zone}} - d_{\text{wells}}) / 2$, where $\mathbf{d}$ is the diameter in mm. Resulting values (h) for samples with EN534-C on the substrate *S. agalactiae* CCM 6187 are listed in Table 10 and ones detected on *S. agalactiae* KMB-534 substrate in Table 11. We determined the highest $\mathbf{h}$ value in sample 10 for isolated EN534-C and on the substrate of *Streptococcus agalactiae* CCM 6187 (collection reference strain) (Table 10).

Table 10. Lytic activity of EN534-C protein on cell substrate *S. agalactiae* CCM 6187 determined by diffusion method

| | substrate *S. agalactiae* CCM 6187 | | | |
|---|---|---|---|---|
| | *sample EN534-C | h (mm) | $d_{\text{zones}}$ | $d_{\text{wells}}$ |
| **1** | **1** | 2 | 9 | 5 |
| **2** | **2** | 3 | 11 | 5 |
| **3** | **3** | 4 | 13 | 5 |
| **4** | **4** | 4 | 13 | 5 |
| **5** | **5** | 5 | 15 | 5 |
| **6** | **6** | 5 | 15 | 5 |
| **7** | **PP** | 0 | 0 | 5 |
| **8** | **E1** | 4 | 13 | 5 |
| **9** | **E3** | 4 | 13 | 5 |
| **10** | **E4** | 17 | 39 | 5 |

*SAMPLES:

1. A suspension of cells containing the expressed recombinant protein EN534-C before sonication, 2. Lysate of induced culture after 1 cycle of sonication, 3. Soluble fraction of lysate - supernatant from an aliquot after 1 cycle of sonication, 4. Lysate of induced culture after 2 cycles of sonication, 5. Soluble fraction of lysate - supernatant from an aliquot after 8 cycles of sonication, 6. Lysate of induced culture after 8 cycles of sonication, 7. PP - fraction containing flow-through proteins after loading on an affinity support, 8. E1 - sample from the elution of the recombinant protein EN534-C from affinity support - fraction 1, 9. E3 - sample from the elution of the recombinant protein EN534-C from affinity support - fraction 3, 10. E4 - sample from the elution of the recombinant protein EN534-C from affinity support - fraction 4.

Table 11. Lytic activity of EN534-C protein on cellular substrate *S. agalactiae* KMB -534 determined by diffusion method

| | substrate *S. agalactiae* KMB-534 | | | |
|---|---|---|---|---|
| | **sample | h (mm) | $d_{\text{zones}}$ | $d_{\text{wells}}$ |
| **1** | **1** | 2 | 9 | 5 |
| **2** | **2** | 0 | | 5 |
| **3** | **3** | 4 | 13 | 5 |
| **4** | **4** | 4 | 13 | 5 |
| **5** | **5** | 4 | 13 | 5 |

(continued)

| | **sample | h (mm) | d_zones | d_wells |
|---|---|---|---|---|
| | | | | |

| | | substrate *S. agalactiae* KMB-534 | | |
|---|---|---|---|---|
| | **\*\*sample** | **h (mm)** | **d$_{zones}$** | **d$_{wells}$** |
| 6 | 6 | 4 | 13 | 5 |
| 7 | PP | 0 | 0 | 5 |
| 8 | E1 | 2.25 | 9.5 | 5 |
| 9 | E3 | 5 | 15 | 5 |
| 10 | E4 | 6 | 17 | 5 |
| 11 | EB | 0 | 0 | 5 |

\*\*Samples:
1. A suspension of cells containing the expressed recombinant EN534-C protein before sonication,
2. Lysate of induced culture after 1 cycle of sonication,
3. Soluble fraction of lysate - supernatant from an aliquot after 1 cycle of sonication,
4. Lysate of induced culture after 2 cycles of sonication,
5. Soluble fraction of lysate - supernatant from an aliquot after 8 cycles of sonication,
6. Lysate of induced culture after 8 cycles of sonication,
7. PP - fraction containing flow-through proteins after loading on an affinity support,
8. E1 - sample from the elution of the recombinant protein EN534-C - fraction 1,
9. E3 - sample from the elution of the recombinant protein EN534-C - fraction 3,
10. E4 - sample from the elution of the recombinant protein EN534-C - fraction 4.
11. check - WEQ elution buffer solution

**Example 4** - Determination of lytic activity of EN534-C by optical method on substrate of strain *Streptococcus agalactiae* CCM 6187

The method is based on measuring the decrease of the optical density of the bacterial substrate by the lytic activity of recombinant endolysins and is used to determine the lytic activity of enzymes.

We used cell walls of bacterial strains *Streptococcus agalactiae, Bacillus subtilis, Enterococcus faecalis, Lactobacillus spp.* and *Escherichia coli* XL-1 Blue as a substrate. Cells were cultured in the appropriate media (Table 2) on an I26R shaker at 30°C or 37°C. Bacterial cultures were grown to middle phase of exponential growth (OD$_{600}$ ~ 0.5) and sedimented by centrifugation on an Eppendorf 5810R centrifuge (4000 x g, 10 min, 4°C). The cell sediment was first washed with SM buffer solution and then resuspended in TN buffer solution (Table 7), frozen in liquid nitrogen and stored at - 20 ° C or -80 ° C, or used immediately as a substrate for lytic activity. After thawing, the cells were washed with buffer solution (50 mmol$^{-1}$ Tris-HCl, pH 8.0, 150 mmol.l$^{-1}$ NaCl), centrifuged using an Eppendorf 5810R centrifuge (2 500 x g, 10 min, 4°C) and resuspended in solution A (Table 7) to absorbance: OD$_{600}$ ~ 0.5 - 1. Lytic activity was determined spectrophotometrically in a microtiter 96-well plate in a reaction mixture in a total volume of 200 µl or in a spectrophotometric cuvette in a total volume of 1 ml. In the prepared microtiter plate or cuvette, we applied protein in a volume of 2 µl or 10 µl, respectively, in a final concentration of 0.1 µmol.ml$^{-1}$. We started the reaction by adding 198 µl or 990 µl of the cell substrate suspension. In the negative control solution E was added to instead of diluted protein (Table 7). We measured the absorbance at OD$_{600}$ and 37°C on a Synergy HT Multi-Mode Microplate Reader (BioTek Instruments, USA) every 2 minutes for 60 minutes. We evaluated the measured values as a decrease in absorbance (OD$_{600}$) over the course of the measurement (during 60 minutes). We calculated the relative lytic activity in % according to formula no. 1 (Palmeira de Oliveira et al., 2015). There is a sample in the formula *r* endolysin-containing substrate and sample *c* is a substrate without the addition of endolysin.

$$\left( \frac{(r_{t0} - r_{tn}) - (c_{t0} - c_{tn})}{c_{t0}} \right) * 100$$

$r_{t0}$ = endolysin sample measured at 0 min,
$r_{tn}$ = sample with endolysin at time n,
$c_{t0}$ = endolysin-free control at 0 min,

ctn = control sample measured at time n

\* = multiplication character

Evaluation:

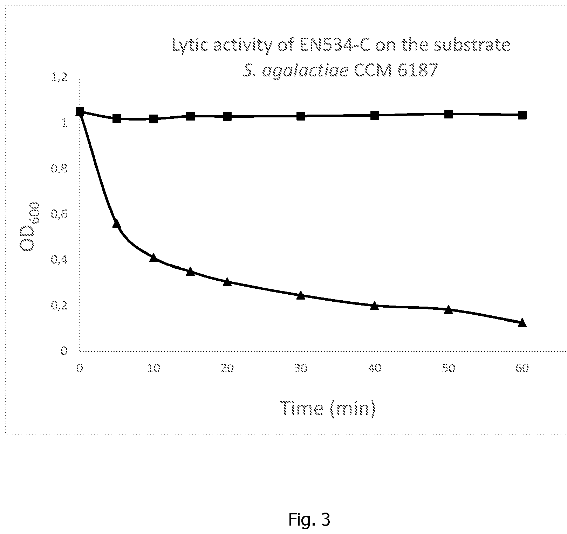

We used an optical method to quantify the lytic activity of the isolated recombinant protein EN534-C. The lytic activity of recombinant endolysin EN534-CHis was measured spectrophotometrically according to the above procedure. Figure 3 shows the course of the lytic activity of recombinant endolysin on the cell wall substrate as a decrease of absorbance during the 60-minute measurement. We recorded the largest decrease of absorbance at the time of measurement from 0 to 10 minutes, where the $OD_{600}$ value decreased from 1.05 to 0.41. The calculated relative lytic activity of the recombinant protein corresponds to 58% in 10 minutes. In 60 minutes, the relative lytic activity of EN534-C reached 90%.

**Example 5** - Antibacterial spectrum of recombinant endolysin EN534-C

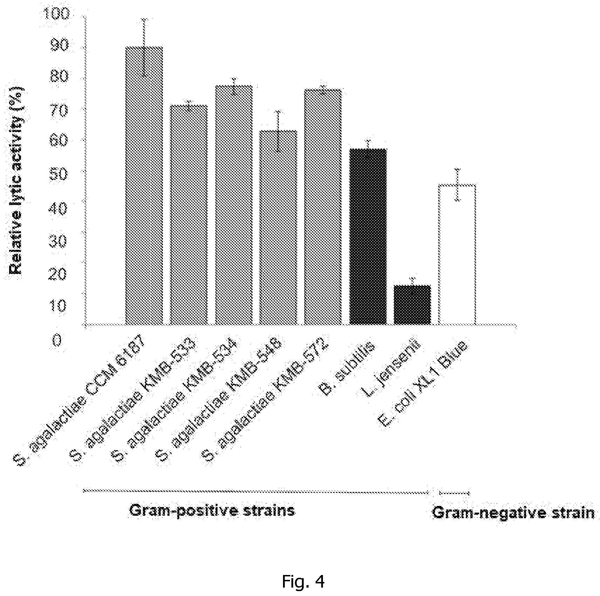

The lytic activity of recombinant endolysin EN534-C against selected bacterial strains represents an antibacterial spectrum. We determined the relative lytic activity of EN534-C using an optical method on the substrates of selected bacterial strains according to the above procedure (Example 4). We selected representatives for Gram-positive bacteria and Gram-negative bacteria. We confirmed the exolytic activity of recombinant endolysin EN534-C on the reference strain *Streptococcus agalactiae* CCM 6187, where the relative lytic activity was highest - 90%. On substrates of human isolates *Streptococcus agalactiae* KMB-533, KMB-534, KMB-548 and KMB-572 (Department of Molecular Biology, Faculty of Sciences, Comenius University) the relative lytic activity values were higher than 60%. The following relative lytic activity was measured on other model bacterial strains: *Bacillus subtilis* 57% and *Escherichia coli* XL1 Blue more than 45 %. On the strain *Lactobacillus jensenii* relative activity of only 15% was measured. *Lactobacillus jensenii* is a human isolate from the vaginal environment, and is a beneficial microflora. The differences in relative lytic activity on different bacterial substrates were shown in a bar graph (Figure 4).

**Example 6** - Fluorescence microscopy

For fluorescence microscopy, *Streptococcus agalactiae* CCM 6187 bacterial cells were cultivated with shaking in 10 ml of THB medium (Table 7) at 37°C and 200 rpm$^{-1}$ to the exponential growth phase $OD_{600}$ ~ 0.5. For visualisation of the membrane structures, we used FM4-64 dye (1 $\mu$g.ml$^{-1}$) (Thermo Fisher Scientific, USA), which we added to an exponentially growing bacterial culture 30 minutes before reaching the desired absorbance ($OD_{600}$ ~ 0.5) and then continued in cultivation. After 30 minutes, 1 ml of cell culture was removed, washed twice with 50 mmol.l$^{-1}$ buffer solution Tris-HCl pH 8.0 and resuspended in 40 $\mu$l in this solution. From the cell suspension thus prepared we took 23 $\mu$l and added 2 $\mu$l SYTOX green (1 $\mu$mol.ml$^{-1}$) (Thermo Fisher Scientific, USA). We added 5 $\mu$l of recombinant endolysin (1 $\mu$mol.ml$^{-1}$) to the prepared cells, mixed, immediately applied 1 $\mu$l to a poly-L-lysine treated slide and covered with a coverslip. We visualized the samples using fluorescence microscopy on an Olympus BX63 instrument with a Hamamatsu Orca-R camera[2]. Images of samples were taken and later processed using Olympus CellP imaging software, Olympus Image-Pro Plus 6.0 or Image J Fiji (shown in Example 1).

Evaluation:

We confirmed the lytic activity of isolated recombinant endolysin EN534-C on living cells by fluorescence microscopy. Bacterial cells *S. agalactiae* CCM 6187 were processed according to the above procedure. The lytic activity of the recombinant protein was visualised using an Olympus BX63 fluorescence microscope with a Hamamatsu Orca-R[2] camera in real time at minute intervals for 6 minutes. Six minutes after the addition of recombinant endolysin, the more than 97% of *S. agalactiae* CCM 6187 cell culture (peptidoglycan lysis) had been lysed (Figure 5).

**Industrial applicability**

The main benefit of the present invention and its applicability in the industrial field is the production of a new preparation or composition suitable for the prevention and treatment of vaginal and urinary tract infections. The isolated protein is thermally stable in the temperature range of 20°C to 37°C and can be isolated at laboratory temperature. This also reduces the energy consumption of the recombinant protein isolation in industrial use. The application of endolysin will expand the possibilities of treating mainly recurrent infections. Its application is also suitable for patients where the use of antibiotics is not recommended. The use of the EN534-C endolysin preparation or composition will make it possible to treat infections caused by both susceptible and resistant bacterial strains. Antibiotic consumption is reduced, and the spread of resistant strains is prevented. There are already several commercial preparations based on recombinant endolysins available on the world market, such as: CF-301 (ContraFect) and StaphefektTM (Micreos Human Health), which are effective against *Staphylococcus aureus* and MRSA.

**List of sequences**

SEQ ID NO:1
Nucleotide and amino acid sequence of the EN534 endolysin gene (peg 1392) from Streptococcus agalactiae KMB-534 of size 489 AK and 1467 bp

```
atg gga aaa cat cta gtg att tgt gga cat ggg caa gga cgc act acc tat gat cca gga
Met Gly Lys His Leu Val Ile Cys Gly His Gly Gln Gly Arg Thr Thr Tyr Asp Pro Gly
 1               5                   10                  15                  20

gca gtg aat agt aaa cgt ggc atc aca gaa gcc gga aag gtt cgt gag ttg gct aga ctc
Ala Val Asn Ser Lys Arg Gly Ile Thr Glu Ala Gly Lys Val Arg Glu Leu Ala Arg Leu
21              25                  30                  35                  40

atg tcc aag tac agc ggc aaa aac atc gat tat atc aca gac caa aat gtt tat gat tat
Met Ser Lys Tyr Ser Gly Lys Asn Ile Asp Tyr Ile Thr Asp Gln Asn Val Tyr Asp Tyr
41              45                  50                  55                  60

aag tca ctg gca atc ctt ggt aag ggc tat gac tct gtt acc gag ctt cat ttc aat gcc
Lys Ser Leu Ala Ile Leu Gly Lys Gly Tyr Asp Ser Val Thr Glu Leu His Phe Asn Ala
61              65                  70                  75                  80

ttt aat ggc act gca cga gga acg gaa gtc ctc att caa tct tct ttg acg gct gat aag
Phe Asn Gly Thr Ala Arg Gly Thr Glu Val Leu Ile Gln Ser Ser Leu Thr Ala Asp Lys
81              85                  90                  95                  100

gag gat ttg gct att cta tct gtc ctt agc cgt cat ttc caa aac cgt ggg att aag aaa
Glu Asp Leu Ala Ile Leu Ser Val Leu Ser Arg His Phe Gln Asn Arg Gly Ile Lys Lys
101             105                 110                 115                 120

gtg gat tgg ctc tat aat gcc aac gaa gct aag aac cgt ggg tac act tac cgc ttg gtg
Val Asp Trp Leu Tyr Asn Ala Asn Glu Ala Lys Asn Arg Gly Tyr Thr Tyr Arg Leu Val
121             125                 130                 135                 140

gag att gcc ttt att gat aac gaa gag gac atg act atc ttt gaa aat aag aag gaa gaa
Glu Ile Ala Phe Ile Asp Asn Glu Glu Asp Met Thr Ile Phe Glu Asn Lys Lys Glu Glu
141             145                 150                 155                 160
```

ctg gcc aaa ggt ctc gtc tct gct att acc caa gag gag gtg aag aca gtt gtc tct gcc
Leu Ala Lys Gly Leu Val Ser Ala Ile Thr Gln Glu Glu Val Lys Thr Val Val Ser Ala
161           165           170           175           180

acc ccc agt aag caa gga gga cag ccc cat gct tct acc agc cct gtt tat cac gtt ggg
Thr Pro Ser Lys Gln Gly Gly Gln Pro His Ala Ser Thr Ser Pro Val Tyr His Val Gly
181           185           190           195           200

gat agt gtt cgt gtg ctt ggt cat gcg act cat tac caa acg ggt caa gcg atg gca agt
Asp Ser Val Arg Val Leu Gly His Ala Thr His Tyr Gln Thr Gly Gln Ala Met Ala Ser
201           205           210           215           220

tgg gtc aaa ggt cgc acc tac aaa atc ctc caa gtc aaa gcc gta aac caa tct cgt agt
Trp Val Lys Gly Arg Thr Tyr Lys Ile Leu Gln Val Lys Ala Val Asn Gln Ser Arg Ser
221           225           230           235           240

aag aga gcc tac tta ctt gaa ggg att aca tct tgg gta ctg gaa cag gat gtg gaa gga
Lys Arg Ale Tyr Leu Leu Glu Gly Ile Thr Ser Trp Val Leu Glu Gln Asp Val Glu Gly
241           245           250           255           260

acc agc ctt ggt cat tca gaa cag acc tat acg gct caa aag gga gat agc tat tgg cga
Thr Ser Leu Gly His Ser Glu Gln Thr Tyr Thr Ala Gln Lys Gly Asp Ser Tyr Trp Arg
261           265           270           275           280

att gca cga aaa tct ggg aca acg gtt gat ggt ctt tta gcc ttg aat ggt ttg aag aaa
Ile Ala Arg Lys Ser Gly Thr Thr Val Asp Gly Leu Leu Ala Leu Asn Gly Leu Lys Lys
281           285           290           295           300

acc gat gtc tta aag att ggt caa acc ttg aaa gtc cat aag gcg acg agt tcc atc aaa
Thr Asp Val Leu Lys Ile Gly Gln Thr Leu Lys Val His Lys Ala Thr Ser Ser Ile Lys
301           305           310           315           320

gca gta gca aca agt ctt gcc caa cgc gcg gtt gct tca gca ctg tct aaa gtt ggt cag
Ala Val Ala Thr Ser Leu Ala Gln Arg Ala Val Ala Ser Ala Leu Ser Lys Val Gly Gln
321           325           330           335           340

aaa gtg act gtt cct acc aac cct tat ggt gga caa tgc gtc agt ctg gtg gat aag att
Lys Val Thr Val Pro Thr Asn Pro Tyr Gly Gly Gln Cys Val Ser Leu Val Asp Lys Ile
341           345           350           355           360

gtg cag gag ttg acc gac aag gac atg gca tac acc aat gcc atc gat tgt tta acc aag

Val Gln Glu Leu Thr Asp Lys Asp Met Ala Tyr Thr Asn Ala Ile Asp Cys Leu Thr Lys
361          365          370          375          380

gcg aaa gct aac ggc ttt aca gtc att aag gac gct tgg ggc gtt aac cct aaa gct ggt
Ala Lys Ala Asn Gly Phe Thr Val Ile Lys Asp Ala Trp Gly Val Asn Pro Lys Ala Gly
381          385          390          395          400

gac ttc tat gtt att aag aca gac agt cat cct tat ggt cac atc ggc att tgt atc aca
Asp Phe Tyr Val Ile Lys Thr Asp Ser His Pro Tyr Gly His Ile Gly Ile Cys Ile Thr
401          405          410          415          420

gat tca gat ggt aca agc att gat ggg gtt gag cag aat gtc gat ggc tac tct gac cac
Asp Ser Asp Gly Thr Ser Ile Asp Gly Val Glu Gln Asn Val Asp Gly Tyr Ser Asp His
421          425          430          435          440

aac aag aac ggt atc aat gac caa ctg gaa att ggt ggt ggc ggt att acc cgt cga gtg
Asn Lys Asn Gly Ile Asn Asp Gln Leu Glu Ile Gly Gly Gly Gly Ile Thr Arg Arg Val
441          445          450          455          460

aag cgt gtc tgg atg gca gat ggc tca ctt tat gat gcg act ggc acc gtc aaa ctt gga
Lys Arg Val Trp Met Ala Asp Gly Ser Leu Tyr Asp Ala Thr Gly Thr Val Lys Leu Gly
461          465          470          475          480

aaa gtt atc ggt tgg ttc aga tta gga
Lys Val Ile Gly Trp Phe Arg Leu Gly
481          485          489

SEQ ID NO: 2

Nucleotide and amino acid sequence of recombinant endolysin EN534-C
Domain sequences in AK: Amidase 3: L5 - 1169; binding domain LysM: Y270 - V312; CHAP: G351 - N433;
His-Tag: H499- H504, 504 AK, 1512 bp

atg gga aaa cat cta gtg att tgt gga cat ggg caa gga cgc act acc tat gat cca gga
Met Gly Lys His Leu Val Ile Cys Gly His Gly Gln Gly Arg Thr Thr Tyr Asp Pro Gly
1           5           10          15          20

gca gtg aat agt aaa cgt ggc atc aca gaa gcc gga aag gtt cgt gag ttg gct aga ctc

Ala Val Asn Ser Lys Arg Gly Ile Thr Glu Ala Gly Lys Val Arg Glu Leu Ala Arg Leu

atg tcc aag tac agc ggc aaa aac atc gat tat atc aca gac caa aat gtt tat gat tat
Met Ser Lys Tyr Ser Gly Lys Asn Ile Asp Tyr Ile Thr Asp Gln Asn Val Tyr Asp Tyr

aag tca ctg gca atc ctt ggt aag ggc tat gac tct gtt acc gag ctt cat ttc aat gcc
Lys Ser Leu Ala Ile Leu Gly Lys Gly Tyr Asp Ser Val Thr Glu Leu His Phe Asn Ala

ttt aat ggc act gca cga gga acg gaa gtc ctc att caa tct tct ttg acg gct gat aag
Phe Asn Gly Thr Ala Arg Gly Thr Glu Val Leu Ile Gln Ser Ser Leu Thr Ala Asp Lys

gag gat ttg gct att cta tct gtc ctt agc cgt cat ttc caa aac cgt ggg att aag aaa
Glu Asp Leu Ala Ile Leu Ser Val Leu Ser Arg His Phe Gln Asn Arg Gly Ile Lys Lys

gtg gat tgg ctc tat aat gcc aac gaa gct aag aac cgt ggg tac act tac cgc ttg gtg
Val Asp Trp Leu Tyr Asn Ala Asn Glu Ala Lys Asn Arg Gly Tyr Thr Tyr Arg Leu Val

gag att gcc ttt att gat aac gaa gag gac atg act atc ttt gaa aat aag aag gaa gaa
Glu Ile Ala Phe Ile Asp Asn Glu Glu Asp Met Thr Ile Phe Glu Asn Lys Lys Glu Glu

ctg gcc aaa ggt ctc gtc tct gct att acc caa gag gag gtg aag aca gtt gtc tct gcc
Leu Ala Lys Gly Leu Val Ser Ala Ile Thr Gln Glu Glu Val Lys Thr Val Val Ser Ala

acc ccc agt aag caa gga gga cag ccc cat gct tct acc agc cct gtt tat cac gtt ggg
Thr Pro Ser Lys Gln Gly Gly Gln Pro His Ala Ser Thr Ser Pro Val Tyr His Val Gly

gat agt gtt cgt gtg ctt ggt cat gcg act cat tac caa acg ggt caa gcg atg gca agt
Asp Ser Val Arg Val Leu Gly His Ala Thr His Tyr Gln Thr Gly Gln Ala Met Ala Ser

tgg gtc aaa ggt cgc acc tac aaa atc ctc caa gtc aaa gcc gta aac caa tct cgt agt
Trp Val Lys Gly Arg Thr Tyr Lys Ile Leu Gln Val Lys Ala Val Asn Gln Ser Arg Ser

aag aga gcc tac tta ctt gaa ggg att aca tct tgg gta ctg gaa cag gat gtg gaa gga
Lys Arg Ale Tyr Leu Leu Glu Gly Ile Thr Ser Trp Val Leu Glu Gln Asp Val Glu Gly

acc agc ctt ggt cat tca gaa cag acc tat acg gct caa aag gga gat agc tat tgg cga
Thr Ser Leu Gly His Ser Glu Gln Thr Tyr Thr Ala Gln Lys Gly Asp Ser Tyr Trp Arg

att gca cga aaa tct ggg aca acg gtt gat ggt ctt tta gcc ttg aat ggt ttg aag aaa
Ile Ala Arg Lys Ser Gly Thr Thr Val Asp Gly Leu Leu Ala Leu Asn Gly Leu Lys Lys

acc gat gtc tta aag att ggt caa acc ttg aaa gtc cat aag gcg acg agt tcc atc aaa
Thr Asp Val Leu Lys Ile Gly Gln Thr Leu Lys Val His Lys Ala Thr Ser Ser Ile Lys

gca gta gca aca agt ctt gcc caa cgc gcg gtt gct tca gca ctg tct aaa gtt ggt cag
Ala Val Ala Thr Ser Leu Ala Gln Arg Ala Val Ala Ser Ala Leu Ser Lys Val Gly Gln

aaa gtg act gtt cct acc aac cct tat ggt gga caa tgc gtc agt ctg gtg gat aag att
Lys Val Thr Val Pro Thr Asn Pro Tyr Gly Gly Gln Cys Val Ser Leu Val Asp Lys Ile

gtg cag gag ttg acc gac aag gac atg gca tac acc aat gcc atc gat tgt tta acc aag
Val Gln Glu Leu Thr Asp Lys Asp Met Ala Tyr Thr Asn Ala Ile Asp Cys Leu Thr Lys

gcg aaa gct aac ggc ttt aca gtc att aag gac gct tgg ggc gtt aac cct aaa gct ggt
Ala Lys Ala Asn Gly Phe Thr Val Ile Lys Asp Ala Trp Gly Val Asn Pro Lys Ala Gly

gac ttc tat gtt att aag aca gac agt cat cct tat ggt cac atc ggc att tgt atc aca
Asp Phe Tyr Val Ile Lys Thr Asp Ser His Pro Tyr Gly His Ile Gly Ile Cys Ile Thr

gat tca gat ggt aca agc att gat ggg gtt gag cag aat gtc gat ggc tac tct gac cac
Asp Ser Asp Gly Thr Ser Ile Asp Gly Val Glu Gln Asn Val Asp Gly Tyr Ser Asp His

aac aag aac ggt atc aat gac caa ctg gaa att ggt ggt ggc ggt att acc cgt cga gtg

Asn Lys Asn Gly Ile Asn Asp Gln Leu Glu Ile Gly Gly Gly Gly Ile Thr Arg Arg Val

441        445        450        455        460

aag cgt gtc tgg atg gca gat ggc tca ctt tat gat gcg act ggc acc gtc aaa ctt gga

Lys Arg Val Trp Met Ala Asp Gly Ser Leu Tyr Asp Ala Thr Gly Thr Val Lys Leu Gly

461        465        470        475        480

aaa gtt atc ggt tgg ttc aga tta gga gtc gac aag ctt gcg gcc gca ctc gag cac cac

Lys Val Ile Gly Trp Phe Arg Leu Gly Val Asp Lys Leu Ala Ala Ala Leu Glu His His

481        485        490        495        500

cac cac cac cac

His His His His

501        504

SEQ ID NO: 3

Sequence of primer EN 534_Ami3 F
ataccatgggaaaacatctagtga 24

SEQ ID NO: 4

Sequence of primer EN 534_Ami3 R
cttgtcgactcctaatctgaaccaa 25

**References**

1. Artimova, K. (2016) Streptokokové infekcie urogenitálneho traktu (Streptococcal infections of the urogenital tract). In: Proceedings of the conference Prowazek Days, March 14 and 15, 2016, Wellness Hotel Patince. SPRÁVY KLINICKEJ MIKROBIOLÓGIE (CLINICAL MICROBIOLOGY REPORTS) EV 2992/09 Volume XVI. Number SA/2016, p. 26. ISSN 1338-645X
2. Fischetti, V.A. (2010). Bacteriophage endolysins: a novel anti-infective to control Gram-positive pathogens. International Journal of Medical Microbiology, 300(6), 357-362. doi:10.1016/j.ijmm.2010.04.002
3. Kuroda, A., Sugimoto, Y., Funahashi, T., Sekiguchi, J. (1992). Genetic structure, isolation and characterization of a Bacillus licheniformis cell wall hydrolase. Molecular and General Genetics, 234 (1), 129-137. https://doi.org/10.1007/BF00272354
4. Nelson, D., Schuch, R., Chahales. P., Zhu, S., Fischetti, V.A. (2006). PlyC: a multimeric bacteriophage lysin. PNAS USA, 103(28), 10765-10770. doi: 10.1073/pnas.0604521103
5. Palmeira-De-Oliveira, R., Palmeira-De-Oliveira, A., Martinez-De-Oliveira, J. (2015). New strategies for local treatment of vaginal infections. Advanced Drug Delivery Reviews, 92, 105-122. doi: 10.1016/j.addr.2015.06.008

**Claims**

1. An antimicrobial protein with lytic properties that contains a sequence of the isolated protein according to SEQ ID NO. 1 or a recombinant protein sequence according to SEQ ID NO. 2.

2. The antimicrobial recombinant protein according to claim 1, having pH optimum for lytic activity in the range of pH 4.0 to 8.0.

3.  The antimicrobial recombinant protein of claim 1 or 2, wherein at a temperature in the range of 20°C to 40°C and at a pH in the range of 5.0 to 8.0, it has at least 60% lytic activity on human isolates *Streptococcus agalactiae.*

4.  The antimicrobial recombinant protein of claim 3, which has 60% to 97% lytic activity on human isolates *Streptococcus agalactiae* or bacteria *Streptococcus agalactiae.*

5.  An expression vector comprising the sequence of SEQ ID NO. 2 according to any one of claims 1 to 4.

6.  A method of preparation of the antimicrobial recombinant protein with lytic properties, according to any one of claims 1 to 4, its overproduction and isolation comprising the following steps:

    a) preparation of the pET-EN534-CHis expression plasmid by cloning the amplified PCR fragment with the endolysin gene from prophage 3 from the strain *Streptococcus agalactiae* KMB-534 into the vector pET28a+;
    b) transformation of the host cells *E. coli* BL21 (DE3) by the construct pET28-EN534-CHis;
    c) growth of the cell culture at 37°C with constant shaking until it reached an OD at 600 nm in the range of 0.5 to 0.6;
    d) induction of cell culture with 0.4 mmol.l$^{-1}$ IPTG;
    e) growth of the culture for at least 1 to 4 hours at a temperature of 28°C to 32°C with permanent shaking;
    f) separation of bacterial cells from the medium,
    g) resuspension of the cells in an equilibration solution,
    h) adding a mixture of protease inhibitors in a volume of 10 $\mu$l/1 ml of suspension,
    i) homogenisation of cells using sonication,
    j) separation of the supernatant with the soluble protein fraction by centrifugation,
    k) protein isolation by IMAC at laboratory temperature,
    l) equilibrating the column, loading the fraction with protein, washing, and eluting the protein.

7.  An antimicrobial composition comprising the antimicrobial recombinant protein according to any one of claims 1 to 4 and a pharmaceutically acceptable carrier.

8.  The antimicrobial recombinant protein according to any one of claims 1 to 4 or the antimicrobial composition according to claim 7, for use as a medicament.

9.  The antimicrobial recombinant protein according to any of claims 1 to 4, or the antimicrobial composition according to claim 7, for use in a treatment, prevention or diagnosis of diseases caused by pathogenic bacteria *Streptococcus agalactiae* GBS.

10. The antimicrobial recombinant protein according to any of claims 1 to 4, or the antimicrobial composition according to claim 7, for use in therapy of multidrug-resistant streptococcal infections in pregnant women, neonates, urogenital infections, non-healing wounds and venous ulcers in adults and mastitis and superficial infections in animals.

11. A method of decontamination of streptococci contaminated surfaces comprising a step of contacting the surface with an effective amount of the antimicrobial protein according to claims 1 to 4, wherein said protein has lytic activity against bacteria *Streptococcus agalactiae* at a pH in the range of 4.0 to 8.0.

**Patentansprüche**

1.  Ein antimikrobielles Protein mit lytischen Eigenschaften, das eine Sequenz des isolierten Proteins nach SEQ ID NO. 1 oder eine Sequenz des rekombinanten Proteins nach SEQ ID NO. 2 enthält.

2.  Das antimikrobielle rekombinante Protein nach Anspruch 1, dessen pH-Optimum für die lytische Aktivität im Bereich von pH 4,0 bis 8,0 liegt.

3.  Das antimikrobielle rekombinante Protein nach Anspruch 1 oder 2, wobei es bei einer Temperatur im Bereich von 20 °C bis 40 °C und einem pH-Wert im Bereich von 5,0 bis 8,0 mindestens 60 % lytische Aktivität auf menschliche Isolate von *Streptococcus agalactiae* aufweist.

**4.** Das antimikrobielle rekombinante Protein nach Anspruch 3, das eine lytische Aktivität von 60 % bis 97 % auf menschliche Isolate *Streptococcus agalactiae* oder Bakterien *Streptococcus agalactiae* aufweist.

**5.** Ein Expressionsvektor, der die Sequenz von SEQ ID NO. 2 nach einem der Ansprüche 1 bis 4 umfasst.

**6.** Ein Verfahren zur Herstellung des antimikrobiellen rekombinanten Proteins mit lytischen Eigenschaften nach einem der Ansprüche 1 bis 4, wobei seine Überproduktion und Isolierung die folgenden Schritte umfasst:

a) Herstellung des Expressionsplasmids pET-EN534-CHis durch Klonierung des amplifizierten PCR-Fragments mit dem Endolysin-Gen aus Prophage 3 aus dem Stamm *Streptococcus agalactiae* KMB-534 in den Vektor pET28a+,
b) Transformation der Wirtszellen *E. coli* BL21(DE3) durch das Konstrukt pET28-EN534-CHis,
c) Wachstum der Zellkultur bei 37°C unter ständigem Schütteln, bis sie eine OD bei 600 nm im Bereich von 0,5 bis 0,6 erreicht,
d) Induktion der Zellkultur mit 0.4 mmol.l$^{-1}$ IPTG,
e) Wachstum der Kultur für mindestens 1 bis 4 Stunden bei einer Temperatur von 28°C bis 32°C unter ständigem Schütteln,
f) Abtrennung der Bakterienzellen vom Medium,
g) Resuspension der Zellen in einer Äquilibrierungslösung,
h) Zugabe einer Mischung von Proteaseinhibitoren in einem Volumen von 10 $\mu$l/1 ml der Suspension,
i) Homogenisierung der Zellen mit Ultraschall,
j) Abtrennung des Überstands mit der löslichen Proteinfraktion durch Zentrifugation,
k) Isolierung des Proteins durch IMAC bei Labortemperatur,
l) Äquilibrieren der Säule, Beladen der Fraktion mit Protein, Waschen und Eluieren des Proteins.

**7.** Antimikrobielle Zusammensetzung, umfassend das antimikrobielle rekombinante Protein nach einem der Ansprüche 1 bis 4 und einen pharmazeutisch akzeptablen Träger.

**8.** Das antimikrobielle rekombinante Protein nach einem der Ansprüche 1 bis 4 oder die antimikrobielle Zusammensetzung nach Anspruch 7 zur Verwendung als Arznei mittel.

**9.** Das antimikrobielle rekombinante Protein nach einem der Ansprüche 1 bis 4 oder die antimikrobielle Zusammensetzung nach Anspruch 7 zur Verwendung bei der Behandlung, Prävention oder Diagnose von Krankheiten, die durch pathogene Bakterien *Streptococcus agalactiae* GBS verursacht werden.

**10.** Das antimikrobielle rekombinante Protein nach einem der Ansprüche 1 bis 4 oder die antimikrobielle Zusammensetzung nach Anspruch 7 zur Verwendung bei der Therapie multiresistenter Streptokokkeninfektionen bei schwangeren Frauen, Neugeborenen, urogenitalen Infektionen, nicht heilenden Wunden und venösen Geschwüren bei Erwachsenen und Mastitis und oberflächlichen Infektionen bei Tieren.

**11.** Ein Verfahren zur Dekontamination von mit Streptokokken kontaminierten Oberflächen, umfassend einen Schritt des Inkontaktbringens der Oberfläche mit einer wirksamen Menge des antimikrobiellen Proteins nach den Ansprüchen 1 bis 4, wobei das genannte Protein eine lytische Aktivität gegen Bakterien *Streptococcus agalactiae* bei einem pH-Wert im Bereich von pH 4,0 bis 8,0 aufweist.

**Revendications**

**1.** Une protéine antimicrobienne avec propriétés lytiques contenant une séquence de la protéine isolée selon SEQ ID NO. 1 ou une séquence de protéine recombinante selon SEQ ID NO. 2.

**2.** La protéine recombinante antimicrobienne selon la revendications 1 avec pH optimum pour l'activité lytique dans une plage de pH de 4,0 à 8,0.

**3.** La protéine recombinante antimicrobienne de la revendication 1 ou 2, où à une température entre 20°C et 40°C et avec un pH dans une plage de 5,0 à 8,0, elle a au moins une activité lytique du 60 % sur les isolats humains de *Streptococcus agalactiae.*

4. La protéine recombinante antimicrobienne de la revendication 3 ayant une activité lytique comprise entre 60 % et 97 % sur les isolats humains de *Streptococcus agalactiae* ou bactéries de *Streptococcus agalactiae*.

5. Un vecteur d'expression comprenant la séquence de SEQ ID NO. 2 selon l'une des revendications de 1 à 4.

6. Une méthode de préparation de la protéine recombinante antimicrobienne avec propriétés lytiques selon l'une des revendications de 1 à 4, avec surproduction et isolement comprenant les étapes suivantes :

a) préparation du plasmide d'expression pET-EN534-CHis par clonage du fragment PCR amplifié avec le gène de l'endolysine de prophage 3 de la souche de *Streptococcus agalactiae* KMB-534 dans le vecteur pET28a+ ;
b) transformation de la cellule hôte *E. Coli* BL21 (DE3) par la structure pET28-EN534-CHis ;
c) croissance de la culture cellulaire à 37°C avec agitation constante jusqu'à ce qu'elle atteigne un OD à 600 nm dans la fourchette de 0,5 à 0,6 ;
d) induction d'une culture cellulaire avec IPTG 0,4 mmol.l$^{-1}$ ;
e) croissance de la culture pendant au moins 1 à 4 heures à une température comprise entre 28°C et 32°C avec agitation permanente ;
f) séparation des cellules bactériennes du fluide ;
g) remise en suspension des cellules dans une solution d'équilibrage ;
h) ajout d'un mélange d'inhibiteurs de protéase dans un volume de 10 $\mu$l/1 ml de suspension ;
i) homogénéisation des cellules par sonication ;
j) séparation du surnageant avec la fraction de protéine soluble soluble par centrifugation ;
k) isolement des protéines par IMAC à la température du laboratoire ;
l) équilibrage de la colonne, chargement de la fraction avec la protéine, lavage et élution de la protéine.

7. Une composition antimicrobienne comprenant la protéine recombinante antimicrobienne selon l'une des revendications de 1 à 4 et un support pharmaceutiquement acceptable.

8. La protéine recombinante antimicrobienne selon l'une des revendications de 1 à 4 ou la composition antimicrobienne selon la revendication 7 pour l'utilisation comme médicament.

9. La protéine recombinante antimicrobienne selon l'une des revendications de 1 à 4 ou la composition antimicrobienne selon la revendication 7 pour l'utilisation dans le traitement, la prévention ou le diagnostic de maladies causées par des bactéries pathogènes de *Streptococcus agalactiae* GBS.

10. La protéine recombinante antimicrobienne selon l'une des revendications de 1 à 4 ou la composition antimicrobienne selon la revendication 7 pour l'utilisation dans le traitement des infections streptococciques multirésistantes chez les femmes enceintes, les nouveau-nés, dans les infections urogénitales, les plaies non cicatrisantes et les ulcères veineux chez les adultes, ainsi que dans les cas de mammites et infections superficielles chez les animaux.

11. Une méthode de décontamination des surfaces contaminées par des streptocoques, comprenant une étape de contact de la surface avec une quantité efficace de protéine antimicrobienne selon les revendications de 1 à 4, où ladite protéine a une activité lytique contre les bactéries de *Streptococcus agalactiae* dans une plage de pH de 4,0 à 8,0.

Fig. 1

Fig. 2

Fig. 3

Fig. 4

| | A | B | C |
|---|---|---|---|
| 2 min | | | |
| 6 min | | | |

Fig.5

Amidase 3
>MGKRLVICGHGQGRTTYDPGAVNSKRGITEAGKVRELARLMSKYSGKNIDYITDQNVYDYKSLA

Amidase 3
ILGKGYDSVTELHFNAFNGTARGTEVLIQSSLTADKEDLAILSVLSRHFQNRGIKKVDWLYNANE

Amidase 3
AKNRGYTYRLVEIAFIDNEEDMTIFENKKEELAKGLVSAITQEEVKTVVSATPSKQGGQPHASTS

PVYHVGDSVRVLGHATHYQTGQAMASWVKGRTYKILQVKAVNQSRSKRAYLLEGITSWVLEQDVE

Lysin
GTSLGHSEQTYTAQKGDSYWRIARKSGTTVDGLLALNGLKKTDVLKIGQTLKVNKATSSIKAVAT

CHAP
SLAQRAVASALSKVGQKVTVPTNPYGGQCVSLVDKIVQELTDKDMAYTNAIDCLTKAKANGFTVI

CHAP
KDAWGVNPKAGDFYVIKTDSHPYGHIGICITDSDGTSIDGVEQNVDGYSDHNKNGINDQLEIGGG

GITRRVKRVWMADGSLYDATGTVKLGKVIGWFRLGVDKLAAALEHHHHHH

Fig. 6

Fig. 7

Fig. 8

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 007572602 B1
- EP 1917355 B1
- WO 2009143446 A3
- US 20160145591 A1
- EP 3252155 A1

### Non-patent literature cited in the description

- **ARTIMOVA, K.** Streptokokové infekcie urogenitálneho traktu (Streptococcal infections of the urogenital tract). *Proceedings of the conference Prowazek Days,* 2016, vol. XVI (SA/2016), ISSN 1338-645X, 26
- **FISCHETTI, V.A.** Bacteriophage endolysins: a novel anti-infective to control Gram-positive pathogens. *International Journal of Medical Microbiology,* 2010, vol. 300 (6), 357-362
- **KURODA, A. ; SUGIMOTO, Y. ; FUNAHASHI, T. ; SEKIGUCHI, J.** Genetic structure, isolation and characterization of a Bacillus licheniformis cell wall hydrolase. *Molecular and General Genetics,* 1992, vol. 234 (1), 129-137, https://doi.org/10.1007/BF00272354
- **NELSON, D. ; SCHUCH, R. ; CHAHALES. P. ; ZHU, S. ; FISCHETTI, V.A.** PlyC: a multimeric bacteriophage lysin. *PNAS USA,* 2006, vol. 103 (28), 10765-10770
- **PALMEIRA-DE-OLIVEIRA, R. ; PALMEIRA-DE-OLIVEIRA, A. ; MARTINEZ-DE-OLIVEIRA, J.** New strategies for local treatment of vaginal infections. *Advanced Drug Delivery Reviews,* 2015, vol. 92, 105-122